# EUROPÄISCHE PATENTSCHRIFT

(11) **EP 1 112 265 B1**
(45) Veröffentlichungstag und Bekanntmachung des Hinweises auf die Patenterteilung: **14.01.2004**
(21) Anmeldenummer: 99947387.9
(22) Anmeldetag: 20.09.1999
(51) Int. Cl.: C07D 401/04, C07D 403/04, A61K 31/44, A61K 31/47, A61K 31/505

(54) **2-ARYLALKYLTHIO -IMIDAZOLE, 2-ARYLALKENYL -THIO -IMIDAZOLE UND 2-ARYLALKINYL -THIO -IMIDAZOLE ALS ENTZÜNDUNGS -HEMMSTOFFE UND HEMMSTOFFE DER CYTOKIN -FREISETZUNG**
2-ARYLALKYLTHIO -IMIDAZOLES, 2-ARYLALKENYL -THIO -IMIDAZOLES AND 2-ARYLALKINYL -THIO -IMIDAZOLES AS ANTI -INFLAMMATORY SUBSTANCES AND SUBSTANCES INHIBITING THE RELEASE OF CYTOKINE
2-ARYLALKYLTHIO-IMIDAZOLES, 2-ARYLALCENYL-THIO-IMIDAZOLES ET 2-ARYLALCYNYL-THIO-IMIDAZOLES EN TANT QUE SUBSTANCES ANTI-INFLAMMATOIRES ET SUBSTANCES INHIBANT LA LIBERATION DES CYTOKINES

(30) Priorität: 18.09.1998 DE 19842833
(43) Veröffentlichungstag der Anmeldung: 04.07.2001
(73) Patentinhaber: MERCKLE GMBH, 89143 Blaubeuren (DE)
(72) Erfinder: LAUFER, Stefan, D-89143 Blaubeuren (DE); STRIEGEL, Hans-Günter, D-89134 Blaustein (DE); NEHER, Karola, D-89143 Blaubeuren (DE)
(74) Vertreter: Best, Michael, Dr.
(86) Internationale Anmeldenummer: PCT/EP1999/006945
(87) Internationale Veröffentlichungsnummer: WO 2000/017192

(56) Entgegenhaltungen:
- WO-A-93/14081
- WO-A-95/00501
- WO-A-96/03387
- WO-A-99/03837
- US-A- 4 461 770
- US-A- 4 584 310
- GALLAGHER T F ET AL: "2,4,5-TRIARYLIMIDAZOLE INHIBITORS OF IL-1 BIOSYNTHESIS" BIOORGANIC & MEDICINAL CHEMISTRY LETTERS,GB,OXFORD, Bd. 5, Nr. 11, Seite 1171-1176 XP000568393 ISSN: 0960-894X
- BOEHM ET AL: "1-Substituted 4-Aryl-5-pyridinylimidazoles: A New Class of Cytokine Suppressive Drugs with Low 5-Lipoxygenase and Cyclooxygenase Inhibitory Potency" JOURNAL OF MEDICINAL CHEMISTRY,US,AMERICAN CHEMICAL SOCIETY. WASHINGTON, Bd. 39, Nr. 20, Seite 3929-3937-3937 XP002103149 ISSN: 0022-2623

## Beschreibung

Die vorliegende Erfindung betrifft 2-Arylalkylthio-imidazol-, 2-Arylalkenyl-thio-imidazol- und 2-Arylalkinyl-thio-imidazolderivate, ein Verfahren zu deren Herstellung und Arzneimittel, die diese Imidazolderivate enthalten.

Es ist bekannt, daß verschiedene Imidazolderivate eine antiinflammatorische Aktivität besitzen. U. a. wurden Verbindungen mit 4,5-Di(hetero)arylimidazol-Strukturelementen eingehend untersucht.

So offenbaren das US-Patent 5,656,644 und die WO 93/14081 4-Aryl-5-heteroaryl-imidazolderivate, die an 2-Position mit einer gegebenenfalls substituierten Aryl- oder Heteroarylgruppe substituiert sind und die eine inhibitorische Aktivität auf die Freisetzung von Cytokinen wie IL-1, IL-6, IL-8 und TNF aufweisen.

Das US-Patent 3,940,486 offenbart 4(5)-Phenyl-5(4)-heteroaryl-imidazolderivate, die an 2-Position mit einem Alkyl-, Cycloalkyl- oder Phenylrest substituiert sind. Es werden verschiedene pharmazeutische Wirkungen dieser Verbindungen, wie beispielsweise eine antiinflammatorische Aktivität, genannt.

Das US-Patent 4,585,771 offenbart 4,5-Diphenylimidazolderivate, die an 2-Position mit einem Pyrrolyl-, Indolyl-, Imidazolyl- oder Thiazolylrest substituiert sind. Diese Verbindungen besitzen eine antiinflammatorische und antiallergische Aktivität.

Die US-Patente 4,528,298 und 4,402,960 offenbaren 4,5-Di(hetero)arylimidazol-derivate, die an 2-Position über eine Thio-, Sulfinyl- oder Sulfonylgruppe mit einem Phenyl-, Pyridyl-, N-Oxypyridyl-, Pyrimidinyl-, Thiazolyl- oder Thienylrest substituiert sind. Diese Verbindungen besitzen eine antiinflammatorische und antiallergische Aktivität.

Die US-Patente 4,461,770 und 4,584,310 offenbaren 4(5)-Aryl-5(4)-heteroarylimidazolderivate, die in 2-Position über eine Thio-, Sulfinyl- oder Sulfonylgruppe mit einem substituierten oder unsubstituierten aliphatischen Kohlenwasserstoffrest substituiert sind. Als substituierter oder unsubstituierter aliphatischer Kohlenwasserstoffrest wird beispielsweise eine Phenyl-C₁₋₄-alkylgruppe genannt, worin der Phenylrest mit C₁₋₄-Alkyl, C₁₋₄-Alkoxy, Halogen mit einem Atomgewicht von nicht mehr als 35, Nitro, Amino oder N,N-Di-C₁₋₄-alkylamino substituiert sein kann. Diese Verbindungen besitzen u.a. eine antiinflammatorische Aktivität.

4,5-Diaryl-substituierte Imidazole als Cyclooxygenase-2-inhibitoren sind in der WO 95/00501 offenbart.

Als molekulares Target der 4-(4-Fluorphenyl)-5-(4-pyridyl)-imidazol-Derivate beschreiben Wilson K.P. et al. (Chemistry & Biology (1997), 4, 423-431) und Young P.R. et al. (J. Biol. Chem. (1997), 272, 12116-12121) die in der Signaltransduktion von Entzündungsreizen in einer Phosphorylierungskaskade aktivierte p38-MAP-Kinase (mitogen aktivierbare Kinase), eine Serin-Threonin-Kinase (Cobb, M.H., Goldsmith, E.J., J. Biol. Chem. (1995), 270, 14843-14846). Nach Wilson K.P. et al. und Young P.R. et al. konkurriert das Strukturelement mit ATP um die Bindung an der ATP-Bindungsstelle des Kinasezentrums (vgl. dazu Tong et al. & Pargellis, C.A. Nat. Struct. Biol. 4, (1997) 311-316).

Substituierte imidazolderivate wurden ebenfalls in den Dokumenten WO96/03387, US-A-4584310, US-A-4461770, WO99/03837, in *Bioorg. Med. Chem. Lett.* **5**, 1171-1176 (1995) und in *J. Med. Chem.* **39**, 3929-3937 (1996) genannt.

Andere 1,2-diarylsubstituierte Heteroaromatensysteme zeigen zudem eine hohe Affinität zu Enzymsystemen der Arachidonsäurekaskade, deren Stoffwechselprodukte entscheidenden Einfluß auf das Entzündungsgeschehen ausüben. Es zeigt sich, daß bei geeigneter Wahl der Substituenten, der den Heterocyclus flankierenden Aromaten, eine günstige Kombinationswirkung auf Targets, wie 5-Lipoxygenase, Cyclooxygenase-1 und -2 und p38-MAP-Kinase (TNF-α, IL-1β-Freisetzung) zustande kommt.

Trotz zahlreicher bekannter Verbindungen besteht weiterhin ein Bedürfnis nach Substanzen mit antiinflammatorischer Aktivität, die die Freisetzung verschiedener Cytokine hemmen und als Hemmstoffe der Mediatoren der Arachidonsäurekaskade dienen. Insbesondere besteht ein Bedürfnis nach Verbindungen, die nicht nur auf die im Akutverlauf entzündlicher Erkrankungen maßgeblichen Parameter einwirken (Entzündungsmediatoren), sondern die auch in die für den chronischen Verlauf entscheidenden immunologischen Vorgänge (Cytokinfreisetzung, Expression von Zelloberflächenantigenen) eingreifen können.

Eine Aufgabe der vorliegenden Erfindung besteht in der Zurverfügungstellung solcher Verbindungen.

Es wurde nun überraschend gefunden, daß 4-Heteroaryl-5-phenyl-imidazolderivate, die an 2-Position mit einer Phenylalkylthiogruppe substituiert sind, deren Phenylrest wiederum mit einer Alkylthio-, Alkylsulfinyl-, Alkylsulfonyl-, Sulfonamido- oder Alkylcarbonylgruppe substituiert ist, diese Aufgabe lösen.

Die vorliegende Erfindung betrifft somit eine Verbindung der allgemeinen Formel I: 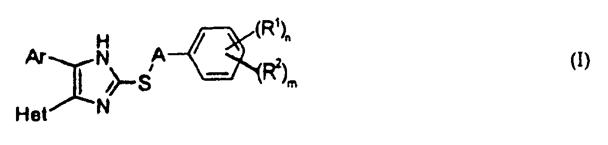 worin
- Ar: für einen Phenylrest steht, der gegebenenfalls mit einem oder mehreren Substituenten, ausgewählt aus Halogen, C₁₋₄-Alkyl, C₁₋₄-Alkoxy und C₁₋₄-Alkylthio, substituiert sein kann;
- Het: für einen Pyridyl-, Pyrimidinyl- oder Pyrazinylrest steht, der gegebenenfalls mit einem oder mehreren Substituenten, ausgewählt aus Halogen, Amino, C₁₋₄-Alkylamino, C₁₋₄-Alkyl, Hydroxy, C₁₋₄-Alkoxy und C₁₋₄-Alkylthio, substituiert sein kann;
- A: für eine geradkettige oder verzweigte, gesättigte oder ungesättigte Alkylenkette mit bis zu 6 Kohlenstoffatomen steht;
- R¹: für C₁₋₄-Alkylthio, C₁₋₄-Alkylsulfinyl, C₁₋₄₋Alkylsulfonyl, Sulfonamido und C₁₋₄-Alkylcarbonyl steht;
- R²: für Halogen, C₁₋₄-Alkyl, Hydroxy, C₁₋₄-Alkoxy, C₁₋₄-Alkoxycarbonyl, Sulfonamido, Carboxyl, Nitro und Aminocarbonyl steht;
- n: 1 oder 2 ist und
- m: 0 bis 2 ist
oder ein pharmazeutisch verträgliches Salz davon.

Unter "Alkyl" wird vorliegend eine niedrige Alkylgruppe mit bis zu 4 C-Atomen verstanden, die geradkettig oder verzweigt sein kann. Hierzu gehören beispielsweise Methyl, Ethyl, Propyl, iso-Propyl, Butyl, iso-Butyl und tert-Butyl.

Unter "Alkoxy", "Alkylthio", "Alkylamino", "Alkylsulfinyl", "Alkylsulfonyl", "Alkylcarbonyl" und "Alkoxycarbonyl" wird vorliegend jeweils eine Gruppe verstanden, die eine vorstehend definierte Alkylgruppe enthält.

Unter "Halogen" wird vorliegend Fluor, Chlor, Brom und Jod verstanden, bevorzugt Fluor, Chlor und Brom und insbesondere Fluor.

Unter einer "geradkettigen oder verzweigten, gesättigten oder ungesättigten Alkylenkette mit bis zu 6 Kohlenstoffatomen" wird vorliegend eine C₁₋₆-Alkylenkette, wie beispielsweise Methylen, Ethylen, 1,3-Propylen, 1-Methyl-ethylen, 2-Methyl-ethylen, 1,4-Butylen, 1-Methyl-1,3-propylen, 2-Methyl-1,3-propylen, 3-Methyl-1,3-propylen, 1-Ethyl-ethylen, 2-Ethyl-ethylen, 2,3-Dimethyl-ethylen und 2,2-Dimethyl-1,3-propylen, eine C₃₋₆-Alkenylenkette mit einer oder mehreren Doppelbindungen; wie beispielsweise Propenylen und Allenylen, und eine C₃₋₆-Alkinylenkette mit einer oder mehreren Dreifachbindungen, wie Propinylen und Butinylen, verstanden. Bei der geradkettigen oder verzweigten, gesättigten oder ungesättigten Alkylenkette A handelt es sich bevorzugt um eine geradkettige, gesättigte Alkylenkette mit 1 bis 4 Kohlenstoffatomen, bevorzugt 1 bis 2 Kohlenstoffatomen, nämlich Methylen oder Ethylen.

Der heteroaromatische Rest Het in der Verbindung der allgemeinen Formel I ist Pyridyl, Pyrimidinyl und Pyrazinyl, wobei diese heteroaromatischen Reste gegebenenfalls substituiert sein können. Bei den Substituenten handelt es sich bevorzugt um ein Halogen, oder eine Aminogruppe. Besonders bevorzugt steht der heteroaromatische Rest Het für 4-Pyridyl, 3-Aminopyridyl, 2,4-Pyrimidinyl und 3-Amino-2,4-pyrimidinyl.

Bei den Substituenten, mit denen der Phenylrest Ar in der Verbindung der allgemeinen Formel I jeweils substituiert sein kann, handelt es sich bevorzugt um Fluor, Chlor, Brom, C₁₋₄-Alkyl, C₁₋₄-Alkoxy und C₁₋₄-Alkylthio. Insbesondere vorteilhaft ist der Phenylrest Ar in para-Stellung mit Fluor, Methoxy oder Methylthio substituiert.

Besonders bevorzugt ist der Phenylrest Ar eine 4-Fluorphenyl-Gruppe.

Die Phenylgruppe des Phenylalkylthio-Restes, der die Imidazolgrundeinheit in der Verbindung der allgemeinen Formel I in 2-Position substituiert, ist erfindungsgemäß mit mindestens einer Gruppe R¹, maximal jedoch mit zwei Gruppen R¹ substituiert. Bei dem Substituenten R¹ handelt es sich um eine C₁₋₄-Alkylthio-, C₁₋₄-Alkylsulfinyl-, C₁₋₄-Alkylsulfonyl-, Sulfonamido- und C₁₋₄-Alkylcarbonylgruppe, wobei R¹ vorteilhaft eine Methylthio-, Methylsulfinyl-, Methylsulfonyl-, Sulfonamido- oder Acetylgruppe bedeutet.

Die Phenylgruppe des Phenylalkylthio-Restes, der die Imidazolgrundeinheit in der Verbindung der allgemeinen Formel I in 2-Position substituiert, kann weitere Substituenten R² umfassen. Bei diesen Substituenten R² handelt es sich um C₁₋₄-Alkyl, C₁₋₄-Alkoxy, C₁₋₄-Alkoxycarbonyl, Sulfonamido, Carboxyl, Hydroxy, Nitro, und Aminocarbonyl sowie Halogen, wie Fluor, Chlor Brom und Jod. Der Phenylrest in der Phenylalkylseitenkette der Verbindung der allgemeinen Formel I kann von 0 bis 2 Substituenten R² aufweisen, die gleich oder verschieden sein können. Bevorzugt sind dabei Verbindungen der allgemeinen Formel I, in denen n 1 und m 0 - 2 ist.

Als besonders vorteilhaft haben sich Verbindungen der allgemeinen Formel I erwiesen, worin der Phenylrest Ar eine 4-Fluorphenylgruppe ist, der heteroaromatische Rest Het eine 4-Pyridyl-, 3-Aminopyridyl-, 2,4-Pyrimidinyl- oder 3-Amino-2,4-pyrimidinylgruppe ist, A für Methylen oder Ethylen steht, n 1 ist und m 0 - 2 ist.

Beispielhaft seien die folgenden Verbindungen der allgemeinen Formel I genannt:
5-(4-Fluorphenyl)-2-[(4-methylthio-phenyl)-methylthio]-4-pyridyl-imidazol
5-(4-Fluorphenyl)-2-[(4-methylsulfinyl-phenyl)-methylthio]-4-pyridyl-imidazol
5-(4-Fluorphenyl)-2-[(4-methylsulfonyl-phenyl)-methylthio]-4-pyridyl-imidazol
2-[(4-Aminosulfonyl-phenyl)-methylthio]-5-(4-fluorphenyl)-4-pyridyl-imidazol
2-[2-(4-Aminosulfonyl-phenyl)-ethylthio]-5-(4-fluorphenyl)-4-pyridyl-imidazol
5-(4-Fluorphenyl)-2-[2-(4-methylthio-phenyl)-ethylthio)-4-pyridyl-imidazol
5-(4-Fluorphenyl}-2-[2-(4-methylsulfonyl-phenyl)-ethylthio]-4-pyridyl-imidazol
5-(4-Fluorphenyl)-2-[(3-methylthio-phenyl)-methylthio]-4-pyridyl-imidazol
5-(4-Fluorphenyl)-2-[(2-methylthio-phenyl)-methylthio]-4-pyridyl-imidazol
5-(4-Fluorphenyl)-2-[(3-methylsulfinyl-phenyl)-methylthio]-4-pyridyl-imidazol
5-(4-Fluorphenyl)-2-[(2-methylsulfinyl-phenyl)-methylthio]-4-pyridyl-imidazol
5-(4-Fluorphenyl)-2-[(4-hydroxy-3-methylthio-phenyl)-methylthio]-4-pyridyl-imidazol
5-(4-Fluorphenyl)-2-[(4-hydroxy-3-methylthio-phenyl)-methylthio)-4-pyridyl-imidazol
2-[(5-Chlor-2-hydroxy-3-methylthio-phenyl)-methylthio]-5-(4-fluorphenyl)-4-pyridyl-imidazol
2-[(5-Chlor-2-hydroxy-3-methylsulfinyl-phenyl)-methylthio]-5-(4-fluorphenyl)-4-pyridyl-imidazol

Es sollte beachtet werden, daß bei den erfindungsgemäßen Verbindungen folgendes Strukturgleichgewicht vorliegt:

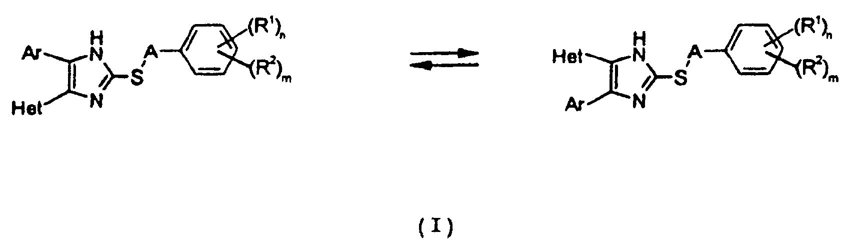

Auch wenn in der Beschreibung und in den Ansprüchen zum leichteren Verständnis nur die 5-Aryl-4-heteroarylimidazolderivate der allgemeinen Formel I beschrieben sind, so umfaßt die vorliegende Erfindung daher auch die 4-Aryl-5-heteroaryl-imidazolderivate

Die vorliegende Erfindung betrifft auch ein Verfahren zur Herstellung einer Verbindung der allgemeinen Formel I, worin ein Imidazol-2-thion der allgemeinen Formel II 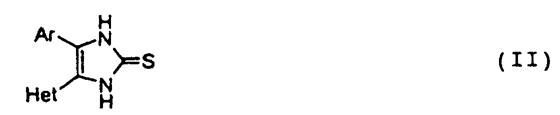 worin Ar und Het wie oben definiert sind, mit einer Verbindung der allgemeinen Formel III 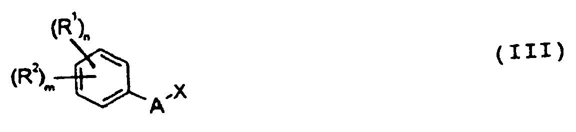 worin A, R¹, R², n und m wie oben definiert sind und X eine Abgangsgruppe ist, zu einer Verbindung der allgemeinen Formel I oder einem pharmazeutisch verträglichen Salz davon umgesetzt wird.

In diesem Verfahren wird die erfindungsgemäße Verbindung in einer nukleophilen Substitutionsreaktion aus entsprechenden Aralkyl-, Aralkenyl- bzw. Aralkinyl-Vorläufern der Formel III und den 5-Aryl-4-heteroaryl-imidazol-2-thionen der Formel II in An- oder Abwesenheit verschiedener Basen, wie Natriumhydrid, Alkalihydroxid, -carbonat oder -acetat hergestellt.

Die Vorläufer der Formel III umfassen eine Abgangsgruppe X, wobei es sich beispielsweise um Chloride, Bromide, Jodide, Acetate oder die Methansulfonsäure-, Toluolsulfonsäure-, und Trifluormethansulfonsäureester der entsprechenden Alkohole, oder weitere dem Fachmann als geeignet bekannte Abgangsgruppen handeln kann.

Als Lösemittel dienen sowohl dipolar aprotische Lösemittel, insbesondere DMF, als auch protische, wie Alkohole, insbesondere Ethenol auch in Mischungen mit Ethern, wie THF.

Eine besonders bevorzugte Ausführungsform des Verfahrens ist die Umsetzung der Aralkyl-, Aralkylenyl-, bzw. Aralkinyl-Vorläufer mit 5-(4-Fluorphenyl)-4-(4-pyridyl)-imidazol-thion (CAS Reg. Nr. 72882-75-8) in Ethanol/THF in Anwesenheit von Natriumcarbonat oder Natriumacetat bei einer Temperatur von 20 - 80 °C.

Zum Beispiel werden 5-(4-Fluorphenyl)-2-[2-(methylsulfinyl)-benzylthio]-4-(4-pyridinyl)-1H-imidazole und 5-(4-Fluorphenyl)-2-[4-(methylsulfinyl)-benzylthio]-4-(4-pyridinyl)-1H-imidazole durch basisch katalysierte Substitution der stellungsisomeren (Methylsulfinyl)-benzylchloride mit 5-(4-Fluorphenyl)-4-(4-pyridyl)-1H-imidazol-2-thion erhalten.

3-(Methylsulfinyl)-benzylchlorid bildet unter denselben Bedingungen kein 5-(4-Fluorphenyl)-2-[3-(methylsulfinyl)-benzylthio]-4-(4-pyridinyl)-1H-imidazols. Es wird durch selektive Oxidation des 5-(4-Fluorphenyl)-2-[3-(methylthio-benzylthio]-4-(4-pyridinyl)-1H-imidazol mit H₂O₂ in Eisessig hergestellt, das durch Substitution des 3-(Methylthio)-benzylchlorids durch 5-(4-Fluorphenyl)-4-(4-pyridyl)-1H-imidazol-2-thion dargestellt werden kann.

Unterschiede bestehen auch in der Syntheseroute für die verschiedenen Vorstufen bei den Methylthio- und Methylsulfinyl-Verbindungen. So erhält man p-Methylsulfinyl-benzylchlorid aus p-Methylthio-benzylalkohol durch Chlorierung und S-Oxidation. Das o-Methylsulfinyl-benzylchlorid ist zugänglich über den S-Methylether der Thiosalicylsäure, der durch LiAlH₄ zum Benzylalkohol reduziert, mit Thionylchlorid chloriert und mit H₂O₂ in Eisessig am S-Atom oxidiert wird. m-Methylthio-benzylchlorid läßt sich ausgehend von Benzoesäure ebenfalls durch Chlorsulfonierung, S-Reduktion, S-Methylierung, C-Reduktion und Chlorierung darstellen. Die Herstellung der übrigen Zwischenstufen der Formel III erfolgt nach herkömmlichen, dem Fachmann bekannten Methoden.

Als Sonderfall lassen sich 2-Hydroxybenzylthio-5-(4-fluorphenyl)-4-(4-pyridinyl)-1H-imidazole der allgemeinen Formel I (R² = OH) auch durch sauer katalysierte Substitution aus geeigneten Hydroxymethylphenolen und 5-(4-Fluorphenyl)-4-(4-pyridinyl)-1H-imidazol-2-thion darstellen. Entsprechende Alkylthio-hydroxymethylphenole der Formel III werden erhalten aus Phenolcarbonsäureestern durch Chlorsulfonierung, S-Reduktion, S-Alkylierung und abschließende C-Reduktion. Die auf diese Weise dargestellten (Alkylthio)benzylthio-imidazole lassen sich mit H₂O₂/Eisessig selektiv zu den (Alkylsulfinyl)benzylthio-imidazolen oxidieren.

Auch das in dem Verfahren der vorliegenden Erfindung als Edukt eingesetzte Imidazol-2-thion der allgemeinen Formel II kann durch herkömmliche, dem Fachmann bekannte Methoden hergestellt werden.

Beispielsweise kann die Herstellung nach dem von I. Lantos et al. (J. Med. Chem. 1984, 27, 72-75) beschriebenen Weg durchgeführt werden. Nach dieser Methode werden Cyanhydrin-Benzoate, die nur in sehr geringen Ausbeuten aus aromatischen Aldehyden zu erhalten sind, mit einem zweiten, aromatischen Aldehyd zu unsymetrischen Benzoinen kondensiert und diese schließlich mit Thioharnstoff zu den Imidazol-2-thionen ringgeschlossen.

Nach einer anderen von Bender et al. in EP 0 231 622 A2 beschriebene Methode wird der Ringschluß mit Azirenen ausgeführt, die in situ mit Rhodanwasserstoff zu den gewünschten Imidazo-2-thionen addieren.

Die letztgenannte Methode wird vorliegend im Beispiel 1.I näher erläutert.

Die erfindungsgemäßen Verbindungen der Formel I zeigen in vivo eine antiinflammatorische Aktivität, sowie in vitro eine Hemmung der Freisetzung verschiedener Cytokine, und sie eignen sich als Inhibitoren der Arachidonsäurekaskade. Sie eignen sich somit zur Behandlung von Erkrankungen, bei denen erhöhte Freisetzungsraten von Cytokinen bzw. der Eicosanoid-Mediatoren für die Entstehung bzw. den progredienten Verlauf dieser Erkrankungen verantwortlich sind.

Die vorliegende Erfindung umfaßt somit auch Arzneimittel, die eine Verbindung der allgemeinen Formel I oder ein pharmazeutisch verträgliches Salz davon sowie gegebenenfalls übliche Trägerstoffe und Hilfsstoffe enthalten.

Weiterhin eignen sich die erfindungsgemäßen Verbindungen der allgemeinen Formel I insbesondere auch zur Herstellung von Arzneimitteln zur Behandlung von Erkrankungen, bei denen die erhöhte Freisetzungsrate von Cytokinen, wie IL-1b und TNF-α, bzw. der Eicosanoid-Mediatoren, wie Hydroperoxyeicosateraensäuren (HPETEs) und Hydroxyeicosateraensäuren (HETEs), Leukotrienen als Produkte des 5-Lipoxygenase-Stoffwechselweges und Prostaglandinen als Produkte des Cyclooxygenase-(1 / 2)-Stoffwechselweges, für die Entstehung bzw. den progredienten Verlauf der Erkrankungen verantwortlich sind. Insbesondere eignen sich die erfindungsgemäßen Verbindungen der allgemeinen Formel I zur Herstellung von Arzneimitteln mit entzündungshemmender Wirkung.

Bevorzugt werden die erfindungsgemäßen Verbindungen der allgemeinen Formel I zur Herstellung von Arzneimitteln zur Behandlung folgender Erkrankungen verwendet:

Rheumatoide Arthritis, rheumatoide Spondylitis, Osteoarthritis, Gicht, multiple Sklerose, toxisches Schocksyndrom, Sepsis, Adult Respiratory Distress Syndrom (ARDS), Inflammatory Bowel Disease (IBD), Cachexie, AIDS Related Complex (ARC), Colitis ulcerosa, Morbus Crohn, entzündliche Hauterkrankungen und psoriatische Arthritis.

Die folgenden Beispiele sollen die vorliegende Erfindung näher erläutern.

### Beispiel 1

### I) Zwischenverbindung

### 5-(4-Fluorphenyl)-4-(4-pyridyl)imidazol-2-thion

### a) 2-Cyano-2-(4-fluorphenyl)-1-(4-pyridyl)ethen-1-ol-hydrochlorid

17,3 g (0,75 mol) metallisches Natrium wurden tropfenweise mit 250 ml absolutem Ethanol versetzt. Dem Ethanolat wurden 75,8 g (0,5 mol) Isonicotinsäureethylester und 67,6 g (0,5 mol) 4-Fluorphenylacetonitril hinzugefügt. Das Reaktionsgemisch wurde 15 min bei 100°C gerührt. Anschließend wurde der Ansatz im Eisbad abgekühlt und mit 600 ml dest. Wasser versetzt. Beim Ansäuern auf pH 1 mit 90 ml konz. HCl fiel die Titelverbindung als gelber Niederschlag aus. Der Niederschlag wurde abfiltriert, mit dest. Wasser gewaschen und im Vakuum über P₂O₅ getrocknet. Ausbeute: 85,0 g (62%).
¹H NMR ([D₆]DMSO/CDCl₃) δ (ppm): 8,8 (AA',2H,4-Pyridyl-), 7,8 (m,2H,4-F-Phenyl), 7,7 (BB',2H,4-Pyridyl-), 7,1 (m,2H,4-F-Phenyl), Enol-Signal nicht sichtbar.

### b) 2-(4-Fluorphenyl)-1-(4-pyridyl)ethanon

40,6 g (0,15 mol) 2-Cyano-2-(4-fluorphenyl)-1-(4-pyridyl)-ethen-1-ol-hydrochlorid wurden in 130 ml 48%iger Bromwasserstoffsäure 19 h unter starkem Rühren refluxiert. Das Reaktionsgemisch wurde im Eisbad abgekühlt, der sich abscheidende Niederschlag (4-Fluorphenylessigsäure) abfiltriert und mit dest. Wasser gewaschen. Beim Neutralisieren des Filtrats mit 80 ml Ammoniakwasser fiel die Titelverbindung als dunkelgrüner Niederschlag aus. Der Niederschlag wurde abfiltriert, mit dest. Wasser gewaschen und im Vakuum über P₂O₅ getrocknet: hellgrau-beiges Pulver. Ausbeute: 14,2 g (45%).
¹H NMR (CDCl₃) δ (ppm): 8,8 (AA',2H,4-Pyridyl-), 7,8 (BB',2H,4-Pyridyl-); 7,2 (m,2H,4-F-Phenyl-), 7,0 (m,2H,4-F-Phenyl-), 4,3 (s,1H,-CH₂-).

### c) 2-(4-Fluorphenyl)-1-(4-pyridyl)ethanonoxim

21,5 g (0,1 mol) 2-(4-Fluorphenyl)-1-(4-pyridyl)ethanon wurden in 330 ml wäßrigem Methanol 50% suspendiert. Nach Zugabe von 36,1 g (0,44 mol) Natriumacetat und 22,0 g (0,32 mol) Hydroxylaminhydrochlorid wurde das Reaktionsgemisch 1 h unter Rühren refluxiert. Beim Abkühlen im Eisbad fiel die Titelverbindung als beigefarbener Niederschlag aus. Der Niederschlag wurde abfiltriert, mit dest. Wasser gewaschen und im Vakuum über P₂O₅ getrocknet. Ausbeute: 14,3 g (62%).
¹H NMR (CDCl₃) δ (ppm): 11,7 (s,1H,Oxim-OH) 8,6 (AA',2H,4-Pyridyl-), 7,5 (BB',2H,4-Pyridyl-), 7,2 (m,2H,4-F-Phenyl-), 6,9 (m,2H,4-F-Phenyl-), 4,1 (s,2H,-CH₂-).

### d) 2-(4-Fluorphenyl)-1-(4-pyridyl)ethanon, O-[(4-methylphenyl)sulfonyl]oxim

In Argonatmosphäre wurden 10,1 g (0,04 mol) 2-(4-Fluorphenyl)-1-(4-pyridyl)ethanonoxim in 50 ml absolutem Pyridin gelöst. Die Lösung wurde auf 6°C abgekühlt und tropfenweise mit 10,1 g (0,05 mol) Toluolsulfonsäurechlorid versetzt. Nach beendeter Zugabe wurde das Reaktionsgemisch 20 h bei Raumtemperatur gerührt. Anschließend wurde der Ansatz auf 500 ml Eiswasser gegossen. Der ausfallende Niederschlag wurde abfiltriert, mit dest. Wasser kalt gewaschen und im Trockenschrank bei 50°C getrocknet. Ausbeute: 14,9 g (88%).
¹H NMR (CDCl₃) δ (ppm): 11,7 (s,1H,Oxim), 8,6 (d,2H,AA'4-Pyridyl-), 7,9 (AA',2H,4-Tosyl-), 7,5 (BB',2H,4-Pyridyl-), 7,4 (BB',2H,4-Tosyl-), 6,9-7,1 (m,4H,4-F-Phenyl-), 4,1 (s,2H, - CH₂-), 2,5 (S,1H,-CH₃).

### e) 5-(4-Fluorphenyl)-4-(4-pyridyl)imidazol-2-thion

In Argonatmosphäre wurden 10,0 g (0,03 mol) 2-(4-Fluorphenyl)-1-(4-pyridyl)ethanon, 0-[(4-methylphenyl)sulfonyl]oxim in 56 ml absolutem Ethanol gelöst. Die Lösung wurde auf 5°C abgekühlt und tropfenweise mit frisch hergestelltem Natriumethanolat aus 0,75 g (0,03 mol) metallischem Natrium in 30 ml absolutem Ethanol versetzt. Das Reaktionsgemisch wurde 5 h bei 5°C gerührt. Nach Zugabe von 500 ml Diethylether wurde weitere 30 min gerührt. Der ausfallende Niederschlag wurde abfiltriert und 4x mit je 50 ml Diethylether gewaschen. Die vereinigte etherische Phase wurde 3x mit je 90 ml 10%iger Salzsäure extrahiert. Der wäßrige Extrakt wurde auf 40 ml eingeengt und mit 5,0 g (0,05 mol) Kaliumthiocyanat versetzt. Das Reaktionsgemisch wurde 1 h unter Rühren refluxiert. Beim Neutralisieren mit 270 ml Natriumhydrogencarbonat-Lösung 5% fiel die Titelverbindung als beiger Niederschlag aus. Der Niederschlag wurde abfiltriert, mit dest. Wasser gewaschen und im Trockenschrank bei 60°C getrocknet. Ausbeute: 5,6 g (79%).
¹H NMR ([D₆]DMSO) δ(ppm): 12,76 (AA' , 2H, 2 NH) 8,50 (AA', 2H, 4-Pyridyl), 7,50-7,42 (m, 2H, 4-F-Phenyl), 7,34-7,25 (m, 4H, 4-Pyridyl + 4-F-Phenyl).
¹³C NMR ([D₆]DMSO) δ(ppm): 164,3 162,1 160,0 149,9 135,5 130,8 130,7 126,8 124,5 121,8 120,4 116,2 115,7.
IR (KBr) 1/λ [cm⁻¹): 1602, 1518, 1228, 1161, 1005, 830, 586, 545.

### II) Erfindungsgemäße Verbindung

### 5-(4-Fluorphenyl)-2-[(4-methylthio)benzylthio]-4-(4-pyridyl)-imidazol

5-(4-Fluorphenyl)-4-(4-pyridyl)imidazol-2-thion (343 mg, 1,3 mmol), hergestellt nach dem oben unter I) beschriebenen Verfahren, wurde in 12 ml einer 50%igen Lösung von Ethanol in THF suspendiert und mit 123 mg (1,5 mmol) Natriumacetat versetzt. In diese Vorlage wurden 258 mg (1,5 mmol) (4-Methylthio)benzylchlorid eingetragen. Das Reaktionsgemisch wurde 4 h unter Rühren refluxiert. Der Ansatz wurde filtriert und das Filtrat eingeengt. Der Rückstand wurde mit Ethylacetat kristallisiert und das Kristallisat abfiltriert. Ausbeute: 0,35 g (68%).
¹H NMR ([D₆)DMSO): δ (ppm) 8,46-8,43 (AA', 2H, 4-Pyridyl-), 7,67-7,05 (m, 11H, 4-F-Phenyl-, 4-CH₃S-Phenyl, 4-Pyridyl-, NH_{Im}), 4,33 (s, 2H, -CH₂-), 2,45 (s, 3H, -SCH₃).
IR (KBr) 1/λ=cm⁻¹: 3429, 1606, 1579, 1504, 1424, 1226, 832.

### Beispiel 2

### 5-(4-Fluorphenyl)-2-[(4-methylsulfinyl)benzylthio]-4-(4-pyridyl)imidazol

5-(4-Fluorphenyl)-4-(4-pyridyl)imidazol-2-thion aus Beispiel 1.I (546 mg, 2,0 mmol) wurden in einer Ethanol/THF-Mischung (1:1, 20 ml) suspendiert und mit Natriumacetat (195 mg, 2,4 mmol) versetzt. Nach Eintrag von (4-Methylsulfinyl)benzylchlorid (520 mg, 2,8 mmol) wurde das Reaktionsgemisch während 12 h unter Rühren refluxiert. Der Ansatz wurde filtriert und das Filtrat eingeengt. Der Rückstand wurde mit Ethylacetat kristallisiert, das Kristallisat abfiltriert und sc gereinigt (SiO₂/Methanol). Ausbeute: 65 mg (8%).
¹H NMR ([D_{4]}MeOH): δ (ppm) 8,4 (AA' , 2H, 4-Pyridyl-), 7, 6 (AA' , 2H, 4-CH₃SO-Phenyl-), 7,5 (BB', 2H, 4-CH₃SO-Phenyl-), 7,4-7,3 (m, 4H, 4-Pyridyl-, 4-F-Phenyl-), 7,2 (m, 2H, 4-F-Phenyl-), 7,4-7,1 (m, 11H, 4-F-Phenyl-, 4-CH₃S-Phenyl, 4-Pyridyl-, NH_{Im}), 4,3 (s, 2H, -CH₂-), 2,7 (s, 3H, -SOCH₃).
IR (KBr) 1/λ=cm⁻¹: 3426, 1602, 1506, 1226, 1157, 1039, 1007, 835, 582.

### Beispiel 3

### 5-(4-Fluorphenyl)-2-[4-methylsulfonyl)benzylthio]-4-(4-pyridyl)imidazol

5-(4-Fluorphenyl)-4-(4-pyridyl)imidazol-2-thion aus Beispiel 1.I (343 mg, 1,3 mmol) wurden in einer Mischung von Ethanol (6 ml) und THF (6 ml) suspendiert und mit Natriumacetat (123 mg, 1,5 mmol) versetzt. In diese Vorlage wurden (4-Methylsulfonyl)benzylchlorid (306 mg, 1,5 mmol) eingetragen. Das Reaktionsgemisch wurde 5 h unter Rühren refluxiert. Der Ansatz wurde filtriert und das Filtrat eingeengt. Der Rückstand wurde mit Ethylacetat kristallisiert und das Kristallisat abfiltriert. Ausbeute: 0,25 g (45%).
¹H NMR ([D₆]DMSO): δ (ppm) 8,4 (AA', 2H, 4-Pyridyl-), 7,9 (AA', 2H, 4-CH₃SO₂-Phenyl-), 7,6 (BB', 2H, 4-CH₃SO₂-Phenyl-), 7,4-7,3 (m, 4H, 4-Pyridyl-, 4-F-Phenyl-), 7,1 (m, 2H, 4-F-Phenyl-), 7,4-7,1 (m, 11H, 4-F-Phenyl-, 4-CH₃S-Phenyl, 4-Pyridyl-, NH_{IM}), 4,4 (s, 2H, -CH₂-), 3,0 (s, 3H, -SCH₃).
IR (KBr) 1/λ=cm⁻¹: 3426, 1602, 1575, 1506, 1304, 1147, 833, 768, 525.

### Beispiel 4

### 2-[2-(4-Aminosulfonylphenyl)ethylthio]-5-(4-fluorphenyl)-4(4-pyridyl)imidazol

5-(4-Fluorphenyl)-4-(4-pyridyl)imidazol-2-thion aus Beispiel 1.I (343 mg, 1,26 mmol) wurde in Ethanol/THF (1:1, 12 ml) suspendiert und mit Natriumacetat (123 mg, 1,5 mmol) versetzt. In diese Vorlage wurden 2-(4-Aminosulfonylphenyl)ethylchlorid (282 mg, 1,3 mmol) eingetragen. Das Reaktionsgemisch wurde 5 h unter Rühren refluxiert. Der Ansatz wurde filtriert und das Filtrat eingeengt. Der Rückstand wurde mit Diisopropylether kristallisiert, das Kristallisat abfiltriert und aus Isopropanol umkristallisiert. Ausbeute: 0,32 g (60%).
¹H NMR ([D₆]DMSO): δ (ppm) 8,35-8,32 (d, 2H, arom.); 7,82-7,77 (d, 2H, arom.); 7,52-7,06 (m, 6H, arom.); 7,15-7,06 (t, 2H, arom.); 3,36 (t, 2H, J=7,4 Hz, CH₂); 3,08 (t, 2H, CH₂)
IR (KBr) 1/λ=cm⁻¹: 3347, 3251, 1603, 1505, 1335, 1222, 1158, 831, 586, 540.

### Beispiel 5

### 2-[(4-Aminosulfonylphenyl)methylthio]-5-(4-fluorphenyl)-4-(4-pyridyl)imidazol

5-(4-Fluorphenyl)-4-(4-pyridyl)imidazol-2-thion aus Beispiel 1.I (1,8 g, 7,2 mmol) und (4-Aminosulfonyl)benzylbromid (1,63 g, 6,0 mmol) wurden in Ethanol (120 ml) suspendiert und mit Natriumcarbonat (1,14 g, 10,8 mmol) versetzt. Das Reaktionsgemisch wurde 4 h unter Rühren refluxiert. Der Ansatz wurde kalt filtriert und das Filtrat eingeengt. Der Rückstand wurde mit Ethylacetat kristallisiert, das Kristallisat abfiltriert und aus THF umkristallisiert.
Ausbeute: 1,1 g (35%).
¹H NMR ([D₆]DMSO): δ (ppm) 8,43-8,40 (d, 2H, arom.); 7,81-7,15 (m, arom.); 4,44 (s, 2H, CH₂);
IR (KBr) 1/λ=cm⁻¹: 3431, 3045, 2926, 1604, 1508, 1304, 1145, 835, 528.

### Beispiel 6

### 2-[2-(4-Methylthiophenyl)ethylthio]-5-(4-fluorphenyl]-4-(4-pyridyl)imidazol

5-(4-Fluorphenyl)-4-(4-pyridyl)imidazol-2-thion aus Beispiel 1.I (406 mg, 1,5 mmol) und 2-[(4-Methylthio)phenyl)-ethylchlorid (350 mg, 1,87 mmol) wurden in Ethanol (30 ml) suspendiert und mit Natriumcarbonat (280 g, 2,7 mmol) versetzt. Das Reaktionsgemisch wurde 48 h unter Rühren refluxiert. Der Ansatz wurde kalt filtriert und das Filtrat eingeengt. Der Rückstand wurde mit Ethylacetat kristallisiert, das Kristallisat abfiltriert.
Rohausbeute: 0,3 g (55%). Umkristallisation aus Isopropanol liefert farblose Substanz 0,1 g (18%).
¹H NMR ([D_{6]}DMSO): δ (ppm) 8,42-8,39 (d, 2H, arom.); 7,52-7,12 (m, 10H, arom.); 3,39-3,32 (t, 2H, CH₂); 3,02-2,98 (t, 2H, CH₂); 2,43 (s, 3H, CH₃)
IR (KBr) 1/λ=cm⁻¹: 3424, 3044, 2924, 1603, 1517, 1226, 1002, 842, 831.

### Beispiel 7

### 5-(4-Fluorphenyl)-2-[2-(4-methylsulfonylphenyl)ethylthio]-4-(4-pyridyl)imidazol

5-(4-Fluorphenyl)-4-(4-pyridyl)imidazol-2-thion aus Beispiel 1.I (1,63 g, 6 mmol) und 2-[(4-Methylsulfonyl)phenyl]-ethylchlorid (1,6 g, 7,3 mmol) wurden in Ethanol (120 ml) suspendiert und mit Natriumcarbonat (1,14 g, 10,8 mmol) versetzt. Das Reaktionsgemisch wurde 60 h unter Rühren refluxiert. Der Ansatz wurde kalt filtriert, der Filterrückstand in Wasser suspendiert. Die wasserunlöslichen Bestandteile wurden abgesaugt, getrocknet und aus MeOH umkristallisiert: Ausbeute 0,9 g.

Das Ethanol-Filtrat der Reaktionslösung wurde eingeengt. Der Rückstand dieser Phase wurde mit MeOH kristallisiert, das Kristallisat abfiltriert.
Ausbeute: 0,6 g
Gesamtausbeute: 1,5 g (55%)
¹H NMR ([D₆]DMSO): δ (ppm): 8,42 (s, 2H, arom.); 7,88-7,83 (m, 2H, arom.); 7,56-7,41 (m, 6H, arom.); 7,21-7,17 (t, 2H, arom.); 3,44-3,41 (t, 2H, CH₂); 3,2-3.1 (m, 5h, CH₂, CH₃)
IR (KBr) 1/λ=cm⁻¹: 3435, 3044, 2934, 1605, 1509, 1411, 1302, 1232, 1144, 1087, 1007, 833.

### Beispiel 8

### 5-(4-Fluorphenyl)-2-[(3-methylthio-phenyl)-methylthio]-4-(4-pyridyl)-1H-imidazol

### a) 3-Chlorsulfonyl-benzoesäure

Benzoesäure (30,5 g, 0,25 mol) wird mit Chlorsulfonsäure (10 ml/1,05 mol) versetzt. Unter Rühren wird das Reaktionsgemisch innerhalb von 20 min auf 120°C erwärmt. Zur Vervollständigung der Umsetzung wird 45 min bei 125°C bis zum Ausbleiben der Gasentwicklung gerührt. Das Reaktionsgemisch wird auf Raumtemperatur (RT) abgekühlt und unter Rühren auf 300 ml Eis gegeben. Der cremefarbene Niederschlag wird abfiltriert, mit Eiswasser gründlich gewaschen und getrocknet. Ausbeute: 40,8 g (74%)
¹H-NMR ([D₆]DMSO) δ (ppm): 8,49 (δ variabel, s, 1H, Carboxyl-OH); 8,28-8,24 (m, 1H, C2-H); 7,98-7,88 (m, 2H, C4-H, C6-H); 7,58-7,51 (m, 1H, C5-H)

### b) Dithio-di-m-benzoesäure

Einer Lösung von 3-Chlorsulfonyl-benzoesäure (20 g, 0,091 mol) in 135 ml Ethanol werden 90 ml konz. Salzsäure zugesetzt. In diese Vorlage wird unter Rühren und anfänglicher Eiskühlung innerhalb von 2 h portionsweise Zinkstaub (32 g, 0,49 mol) eingetragen. Nach 30 min wird die Kühlung entfernt und das Reaktionsgemisch bei RT weitergerührt. Nach beendeter Zugabe wird das Reaktionsgemisch weitere 3 h bei RT gerührt. Das Reaktionsgemisch wird filtriert und der Rückstand mit wenig Ethanol gewaschen. Die vereinigten Filtrate werden unter Rühren portionsweise mit festem FeCl₃ versetzt, bis die Lösung eine bleibende braune Färbung annimmt. Beim Stehenlassen bei RT scheidet sich die Titelverbindung innerhalb weniger Minuten als beiger Niederschlag ab. Das Rohprodukt wird abfiltriert, mit H₂O gewaschen und getrocknet. Ausbeute: 7,5 g (54%)
¹H-NMR ([D₆]DMSO) δ (ppm): 8,10-8,03 (m, 2H, C2-H + C2'-H); 7,90-7,74 (m, 4H, C4-H, C6-H, C4'-H, C6'-H); 7,60-7,50 (m, 2H, C5-H, C5'-H); Carboxyl-OH nicht sichtbar

### c) 3-Methylthio-benzoesäure

In eine Lösung von Dithio-di-m-benzoesäure (7,5 g, 25 mmol) in 125 ml 1N-Natronlauge wird Na₂S x 6-9 H₂O (3,1 g, 14 mmol) eingetragen. Das Reaktionsgemisch wird innerhalb von 15 min unter Rühren auf Rückflußtemperatur erwärmt und weitere 60 min unter Rückfluß gerührt. Die hellbraune Suspension wird auf RT abgekühlt und bei 30°C portionsweise mit Dimethylsulfat (5,4 ml/56 mmol) versetzt. Das Reaktionsgemisch wird 2,5 h bei RT und weitere 30 min unter Rückfluß gerührt. Das Reaktionsgemisch wird auf RT abgekühlt, mit 30 ml dest. H₂O ergänzt und mit konz. Salzsäure tropfenweise auf pH=1 angesäuert. Der hellbraune Niederschlag wird abfiltriert, mit H₂O gewaschen und getrocknet. Das Rohprodukt wird aus 300 ml 50%igem wäßrigem Methanol unter Aktivkohle-Zusatz (0,5 g) umkristallisiert und heiß filtriert: silbern-weiße Blättchen, Ausbeute: 3,7 g (44%)
¹H-NMR ([D₆)DMSO): δ (ppm) = 7,78-7,70 (m, 2H, C2-H, C4-H); 7,54-7,41 (m, 2H, C5-H, C6-H); 2,53 (s, 3H, Methyl); Carboxyl-OH nicht sichtbar
¹³C-NMR ([D₆)DMSO): δ (ppm) = 166,84; 138,89; 131,43; 129,98; 129,08; 125,97; 125,58; 14,46

### d) 3-Hydroxymethyl-1-methylthio-benzol

LiAlH₄ 95% (1,6 g, 40 mmol) wird in einem ausgeheizten und mit Argon gespülten 3-Hals-Kolben in 75 ml absolutes THF eingetragen. Unter Eiskühlung wird zu dieser Vorlage eine Lösung von 3-Methylthio-benzoesäure (5,2 g, 31 mmol) in absolutem THF (25 ml) innerhalb von 15 min so zugetropft, daß nur mäßige Gasentwicklung erfolgt. Nach beendeter Zugabe wird die Kühlung entfernt und das Reaktionsgemisch 30 min bei RT und weitere 2 h bei 60-65°C gerührt. Das Reaktionsgemisch wird auf RT abgekühlt und unter Eiskühlung vorsichtig mit Eiswasser versetzt. Der Niederschlag von Al(OH)₃ wird durch Zusatz von 10%iger Schwefelsäure aufgelöst. Die organische Phase wird abgetrennt und die wäßrig-saure Phase 3x mit Diethylether extrahiert. Die vereinigten etherischen Extrakte werden 2x mit gesättigter NaCl-Lösung und 2x mit dest. H₂O gewaschen, über Na₂SO₄ getrocknet und eingeengt. Das ölige Rohprodukt wird durch Destillation am Kugelrohr (2,5x10⁻² mbar, 155-175°C) gereinigt: farbloses Öl, Ausbeute: 4,5 g (94%).
¹H-NMR (CDCl₃): δ (ppm) = 7,32-7,10 (m, 4H, Ph-H); 4,67 (s, 2H, Methylen); 2,49 (s, 3H, Methyl); 1,72 (s, 1H, austauschbar, OH)

### e) 3-Chlormethyl-1-methylthio-benzol

3-Hydroxymethyl-1-methylthio-benzol (3,3 g, 21 mmol) wird unter Argonatmosphäre in absolutem CH₂Cl₂ (20 ml) gelöst. Dieser Vorlage wird innerhalb von 15 min zunächst unter Rückfluß, dann bei 30°C Innentemperatur eine Lösung von SOCl₂ (2,6 g, 21 mmol) in absolutem CH₂Cl₂ (10 ml) unter Rühren zugetropft. Das Reaktionsgemisch wird 2,25 h unter Rückfluß gerührt. Das Reaktionsgemisch wird eingeengt und das ölige Rohprodukt durch Kugelrohrdestillation (7,9x10⁻² mbar, 125-140°C) gereinigt: farbloses Öl; Ausbeute: 3,3 g (88%)
¹H-NMR (CDCl₃): δ (ppm) = 7,28-7,16 (m, 4H, Ph-H); 4,55 (s, 2H, Methylen); 2,49 (s, 3H, Methyl)

### f) 5-(4-Fluorphenyl)-2-[(3-methylthio-phenyl)-methylthio]-4-(4-pyridyl)-1H-imidazol

In Argonatmosphäre wird 3-Chlormethyl-1-methylthio-benzol (690 mg, 4,1 mmol) in 60 ml absolutem Ethanol gelöst. In diese Vorlage wird 5-(4-Fluorphenyl)-4-(4-pyridyl)imidazol-2-thion (1,1 g, 4,1 mmol) eingetragen. Das Reaktionsgemisch wird 11 h unter Rückfluß gerührt. Die Heizung wird entfernt und die orangefarbene Lösung 61 h bei RT nachgerührt. Der gelbe Niederschlag wird abfiltriert und getrocknet. Ausbeute: 1,21 g (73%)
¹H-NMR ([D6]DMSO): δ (ppm) = 8,67 (m, 2H, AA' 4-Pyr); 7,91 (m, 2H, BB' 4-Pyr); 7,63-7,56 (m, 2H, 4-F-Ph); 7,43-7,16 (m, 6H, 4-F-Ph + 3-H₃CS-Ph); 4,46 (s, 2H, Methylen); 2,40 (s, 3H, Methyl); NH nicht sichtbar
¹³C-NMR ([D₆]DMSO): δ (ppm) = 165,05; 160,14; 148,91; 143,33; 141,29; 138,69; 138,15; 136,88; 131,30; 131,13; 130,58; 128,96; 126,21; 125,42; 124,71; 121,44; 116,46; 116,03; 35,95; 14,52

### Beispiel 9

### 5-(4-Fluoriphenyl-2-[(3-methylsulfinyl-phenyl)-methylthio]-4-(4-pyridyl)-1H-imidazol

5-(4-Fluorphenyl)-2-[(3'-methylthio-phenyl]methylthio-4-(4-pyridyl)-1H-imidazol (Beispiel 8, 500 mg, 1,2 mmol) wird in 7 ml Eisessig suspendiert. Der Vorlage wird 35%ige H₂O₂-Lösung (0,13 ml/1,3 mmol) zugetropft. Das Reaktionsgemisch wird, 20,5 h bei RT gerührt. Das Reaktionsgemisch wird mit 5 mL H₂O verdünnt und unter Eiskühlung tropfenweise mit konz. Ammoniakwasser auf pH=9 eingestellt. Der wäßrige Überstand wird abdekantiert und 3x mit Ethylacetat extrahiert. Der ölige Rückstand wird in Ethylacetat aufgenommen. Die organischen Lösungen werden vereinigt, 3x mit gesättigter NaCl-Lösung gewaschen, über Na₂SO₄ getrocknet und eingeengt. Das ölige Rohprodukt wird säulenchromatographisch (sc) gereinigt (RP-18/MeOH): blaßgelbes Pulver, Ausbeute: 156 mg (31%).
IR (KBr) : 1/λ[cm⁻¹] = 3099, 3057, 2937, 1601, 1500, 1418, 1228, 1019, 837, 829, 694;
¹H-NMR ([D₃)MeOD): δ (ppm) = 8,41 (dd, 2H, J=5,0 Hz, J=1,4 Hz, AA' 4-Pyr); 7,58-7,37 (m, 8H, BB' 4-Pyr, 4-F-Ph, 3-H₃CSO-Ph); 7,21-7,13 (m, 2H, 4-F-Ph); 4,37 (s, 2H, Methylen); 2,67 (s, 3H, Methyl);
¹³C-NMR ([D₃]MeOD): δ (ppm) = 167,0; 162,0; 150,2; 149,8; 146,6; 141,4; 133,2; 132,0; 131,9; 130,9; 125,1; 123,9; 123,0; 117,3; 116,9; 43,7; 39,6.

### Beispiel 10

### 5-(4-Fluorphenyl)-2-[(2-methylsulfinyl-phenyl)-methylthio]-4-(4-pyridyl)-1H-imidazol

### a) 2-Methylthio-benzoesäure

Unter Argonatmosphäre wird Thiosalicylsäure (0,5 g, 3,2 mmol) in 3,2 ml 10%iger Natronlauge gelöst. Dieser Vorlage wird bei RT Dimethylsulfat (0,31 ml/3,2 mmol) unter Rühren zugetropft. Das Reaktionsgemisch wird 15 min bei RT und weitere 60 min unter Rückfluß gerührt. Das Reaktionsgemisch wird auf RT abgekühlt und unter Wasserkühlung mit 10%iger Salzsäure auf pH=1 angesäuert. Der weiße Niederschlag wird abfiltriert, mit H₂O gewaschen und getrocknet. Ausbeute: 520 mg (96%).
¹H-NMR ([D₆]DMSO): δ (ppm) = 7,91 (dd, 1H, J=7,8 Hz, J=1,3 Hz, C3-H); 7,56 (ddd, 1H, J=7,7 Hz, J=7,6 Hz, J=1,5 Hz, C5-H); 7,38-1,34 (m, 1H, C6-H); 7,22 (m, 1H, C4-H); 2,40 (s, 3H, Methyl); Carboxyl-OH nicht sichtbar

### b) 2-Hydroxymethyl-1-methylthio-benzol

LiAlH₄ 95% (1,6 g, 40 mmol) wird in einem ausgeheizten und mit Argon gespülten Dreihalskolben in 75 ml absolutes THF eingetragen. Unter Eiskühlung wird zu dieser Vorlage eine Lösung von 2-Methylthio-benzoesäure (5,2 g, 31 mmol) in 25 ml absolutem THF innerhalb von 15 min so zugetropft, daß nur mäßige Gasentwicklung erfolgte. Nach beendeter Zugabe wird die Kühlung entfernt und das Reaktionsgemisch 30 min bei RT und weitere 2 h bei 60-65°C gerührt. Das Reaktionsgemisch wird auf RT abgekühlt vorsichtig mit Eiswasser versetzt. Der Niederschlag von Al(OH)₃ wird durch Zusatz von 10%iger Schwefelsäure aufgelöst. Die organische Phase wird abgetrennt und die wäßrig-saure Phase 3x mit Diethylether extrahiert. Die vereinigten etherischen Extrakte werden 2x mit gesättigter NaCl-Lösung und 2x mit dest. H₂O gewaschen, über Na₂SO₄ getrocknet und eingeengt. Das ölige Rohprodukt wird durch Destillation am Kugelrohr (2,5x10⁻² mbar, 155-175°C) gereinigt: farbloses Öl, Ausbeute: 4,5 g (94%).
¹H-NMR (CDCl₃): δ (ppm) = 7,39-7,36 (m, 1H, C6-H); 7,31-7,14 (m, 3H, C3-H, C4-H, C5-H); 4,76 (s, 2H, Methylen); 2,49 (s, 3H, Methyl); 2,03 (s, 1H, OH)

### c) 2-Chlormethyl-1-methylthio-benzol

2-Hydroxymethyl-1-methylthio-benzol (3,3 g, 21 mmol) wird unter Argonatmosphäre in 20 ml absolutem CH₂Cl₂ gelöst. Dieser Vorlage wird innerhalb von 15 min zunächst unter Rückfluß, dann bei 30°C Innentemperatur eine Lösung von SOCl₂ (2,6 g, 21 mmol) in 10 ml absolutem CH₂Cl₂ unter Rühren zugetropft. Das Reaktionsgemisch wird 2,25 h unter Rückfluß gerührt. Das Reaktionsgemisch wird eingeengt und das ölige Rohprodukt durch Kugelrohrdestillation (7,9x10⁻² mbar, 125-140°C) gereinigt: farbloses Öl; Ausbeute: 3,3 g (88%).
¹H-NMR (CDCl₃): δ (ppm) = 7,40-7,13 (m, 4H, Ph-H); 4,74 (s, 2H, Methylen); 2,51 (s, 3H, Methyl)

### d) 2-Chlormethyl-1-methylsulfinyl-benzol

2-Chlormethyl-1-methylthio-benzol (3,0 g, 17,4 mmol) wird in 35 ml Eisessig gelöst. Dieser Vorlage wird unter Eiskühlung auf 3-8°C eine Lösung von 35%iger H₂O₂-Lösung (2,15 g, 22 mmol) in 10 ml Eisessig innerhalb von 5 min zugetropft. Die Kühlung wird entfernt und das Reaktionsgemisch 8 h bei RT gerührt, wobei nach 6 h bzw. 7 h weitere 35%ige H₂O₂-Lösung (0,15 g, 1,5 mmol bzw. 0,1 g, 1,0 mmol) zugesetzt wird. Das Reaktionsgemisch wird mit Eis versetzt und mit konz. Ammoniakwasser auf pH=4 eingestellt. Der weiße Niederschlag wird abfiltriert, mit H₂O gewaschen und getrocknet. Die wäßrig-saure Lösung wird mit konz. Ammoniakwasser auf pH=7 eingestellt. Der Niederschlag wird abfiltriert, mit H₂O gewaschen und getrocknet. Die wäßrig-saure Lösung wird mit Ethylacetat extrahiert. Der organische Extrakt wird 2x mit 8%iger NaHCO₃-Lösung und 2x mit gesättigter NaCl-Lösung gewaschen, über Na₂SO₄ getrocknet und eingeengt: gelbes Öl, das bei RT langsam kristallisierte. Gesamtausbeute: 3,12 g (95%).
¹H-NMR (CDCl₃): δ (ppm) = 8,07 (dd, 1H, J=7,8 Hz, 1,4 Hz, C6-H); 7,62 (ddd, 1H, J=7,4 Hz, J=7,2 Hz, J=1,6 Hz, C4-H); 7,52 (ddd, 1H, J=7,5 Hz, J=7,3 Hz, J=1,6 Hz, C5-H); 7,43 (dd, 1H, J=7,5 Hz, J=1,6 Hz, C3-H) ; 4,83 (d, 1H, J=11,7 Hz, Methylen); 4,65 (d, 1H, J=11,7 Hz, Methylen); 2,85 (s, 3H, Methyl);
¹³C-NMR (CDCl₃): δ (ppm) = 145,33; 134,34; 131,56; 130,61; 130,52; 124,25; 44,06; 41,64

### f) 5-(4-Fluorphenyl)-2-[(2-methylsulfinyl-phenyl)-methylthio]-4-(4-pyridyl)-1H-imidazol

Unter Schutzgas (Argon) wird 5-(4-Fluorphenyl)-4-(4-pyridyl)-1H-imidazol-2-thion (0,28 g, 1,03 mmol) und 2-Chlormethyl-1-methylsulfinyl-benzol (0,18 g, 0,95 mmol) in EtOH (15 ml) suspendiert und 4 h unter Rückfluß (Innentemperatur (IT) 77°C) erhitzt. Die Mischung färbt sich tieforangerot und klärt sich. Danach wird die Mischung eingeengt und der orangerot gefärbte Rückstand getrocknet (0,48 g). Das Produkt wird in wenig warmem MeOH gelöst und tropfenweise mit Ethylacetat versetzt bis zur beginnenden Abscheidung. Die Kristallisation erfolgt langsam in der Kälte. Ausbeute : 230 mg (54 %).
IR (KBr): 1/λ[cm⁻¹] = 3057, 2979, 2919, 2901, 2625, 1634, 1606, 1556, 1522, 1490, 1470, 1350, 1223, 1213, 1155, 1062, 1033, 969, 843, 812, 740
¹H-NMR ([D₃]MeOD): δ (ppm) = 8,57 (m, 2H, AA' 4-Pyr); 8,01 (m, 2H, BB' 4-Pyr); 7,95 (d, 1H, J=7,2 Hz, C3'-H); 7,62-7,47 (m, 5H, 4-F-Ph, C4'-H, C5'-H, C6'-H); 7,33-7,24 (m, 2H, 4-F-Ph); 4,62 (d, 1H, 2J=13,6 Hz, Methylen); 4,50 (d,1H 2J=13,6 Hz, Methylen); 2,87 (s, 3H, Methyl)
¹³C-NMR ([D₃]MeOD): δ (ppm) = 167,6; 162,7; 152,3; 145,3; 143,8; 142,3; 138,9; 136,7; 133,4; 132,8; 132,5; 132,4; 132,0; 130,7; 126,9; 125,0; 123,5; 117,9; 117,5; 43,5; 35,1

### Beispiel 11

### 5-(4-Fluorphenyl)-2-[(4-hydroxy-3-methylthio-phenyl)-methylthio]-4-(4-pyridyl)-1H-imidazol

### a) 4-Methoxybenzoesäureethylester

Unter Schutzgas wird 4-Hydroxybenzoesäureethylester (15,0 g, 0, 09 mol) in einer Vorlage von KOH (6,3 g, 0,11 mol) in dest. Wasser (60 ml) eingerührt. Zur klaren Lösung wird Dimethylsulfat (8,8 ml, 0,09 mol) unter Rühren und Eiskühlung so zugetropft, daß die Temperatur 15°C nicht überstieg. Nach beendeter Zugabe wird die Kühlung entfernt, das Reaktionsgemisch zur Vervollständigung der Umsetzung 45 min bei RT und 2 h unter Rückfluß gerührt, dann auf RT abgekühlt. Das abgeschiedene Öl wird in Diethylether (50 ml) aufgenommen. Die abgetrennte, wäßrige Phase mit Diethylether (150 ml) extrahiert, die erhaltenen, etherischen Extrakte werden vereinigt, mit Natronlauge (10%ig, 50 ml) und mit gesättigter NaCl-Lösung gewaschen, über Na₂SO₄ sicc. getrocknet und i.Vac. eingeengt: farbloses Öl, Ausbeute: 15,7 g (97%).
¹H NMR ((D₆]DMSO): δ (ppm): 8,00 (2H, J=6,9 Hz, J=2,2 Hz, AA' 4-Methyl-0-Ph); 6,92 (dd, 2H, J=6,9 Hz, J=1,8 Hz, BB' 4-H₃C-O-Ph); 4,35 (q, 2H, J=7,1 Hz, Methylen); 3,86 (s, 3H, O-Methyl); 1,38 (t, 3H, J=7,1 Hz, Methyl).

### b) 3-Chlorsulfonyl-4-methoxy-benzoesäure-ethylester

Eine Lösung von 4-Methoxy-benzoesäure-ethylester (10,25 g, 57 mmol) in CCl₄ (40 ml) wird auf -15°C abgekühlt und innerhalb von 15 min tropfenweise mit Chlorsulfonsäure (10,4 ml, 156 mmol) versetzt, wobei die Temperatur auf -10°C ansteigt. Nach beendeter Zugabe wird das Reaktionsgemisch innerhalb von 30 min unter Rühren auf RT erwärmt und anschließend 1,5 h bei 40-50°C gerührt. Die Heizung wird entfernt und das Reaktionsgemisch zur Vervollständigung der Chlorierung 64 h im schwachen Argonstrom bei RT gerührt. Das Reaktionsgemisch wird unter Eiskühlung und kräftigem Rühren auf eine Suspension von Eis (25 g) in CCl₄ (50 ml) gegeben. Es wird 3 min kräftig gerührt. Die organische Phase wird abgetrennt und die wäßrige Phase noch CH₂Cl₂ (150 ml) dreimal extrahiert. Die vereinigten organischen Extrakte werden mit gesättigter NaCl-Lösung gewaschen, über Na₂SO₄ sicc. getrocknet und eingeengt: es verbleibt ein kristalliner, weißer Rückstand eines Gemisches von Ester und freier Säure im Verhältnis 1:1, das an der Ölpumpe getrocknet wird, Ausbeute 6,7 g (42%)
¹H NMR ([D₆]DMSO) : δ (ppm) : 8, 63 (d, 1H, J=2,1 Hz, C2-H) ; 8,37 (dd, 1H, J=8,8 Hz, J=2,1 Hz, C6-H); 7,19 (d, 1H, J=8,8 Hz, C5-H); 4,41 (q, 2H, J=7,1 Hz, Methylen); 4,14 (s, 3H, O-Methyl); 1,41 (t, 3H, J=7,2 Hz, Methyl)

### c) 4-Methoxy-3-methylthio-benzoesäure

Innerhalb von 10 min wird Ph₃P (20,5 g, 78 mmol) in eine Suspension des unter b) erhaltenen Gemisches von 3-Chlorsulfonyl-4-methoxy-benzoesäure und 3-Chlorsulfonyl-4-methoxy-benzoesäure-ethylester (5,1 g, 19,3 mmol, bezogen auf Massenmittelwert aus Ester und Säure) in 50 ml Toluol portionsweise eingetragen. Nach beendeter Zugabe wird das Reaktionsgemisch 4,5 h bei RT gerührt. Der feinkristalline Niederschlag (Ph₃P-Oxid) wird abfiltriert und mit Toluol gewaschen. Die vereinigten Filtrate werden mit je 30 ml 10%iger Natronlauge (120 ml) 4mal extrahiert. Die wäßrig-alkalischen Extrakte werden vereinigt, mit Dimethylsulfat (2 ml/21 mmol) versetzt, 2 h bei RT gerührt und abschließend auf Siedetemperatur erwärmt. Das Reaktionsgemisch wird auf RT abgekühlt und unter Eiskühlung mit 20%iger Salzsäure auf pH=1 angesäuert. Der weiße Niederschlag wird abfiltriert, mit dest. Wasser gewaschen und getrocknet. Ausbeute 2,8 g (74%)
¹H NMR ([D₃]MeOD): δ (ppm) = 7,86-7,79 (m, 2H, C2-H + C6-H); 6,98 (d, 1H, J=8,4 Hz, C5-H); 3,93 (s, 3H, O-Methyl); 2,43 (s, 3H, S-Methyl)

### d) 4-Hydroxy-3-methylthio-benzoesäure

4-Methoxy-3-methylthio-benzoesäure (0,5 g, 2,5 mmol) wird in 7 ml eines Gemisches von Eisessig und 48%iger HBr (1+1) suspendiert. Das Reaktionsgemisch wird 6 h unter Rückfluß gerührt. Das Reaktionsgemisch wird auf RT abgekühlt und auf 20 ml H₂O gegeben. Die wäßrige Lösung wird mit 10%iger Na₂CO₃-Lösung auf pH=2 eingestellt und 4x mit je 20 ml Diethylether extrahiert. Die organischen Extrakte werden vereinigt, 2x mit gesättigter NaCl-Lösung gewaschen, über Na₂SO₄ getrocknet und eingeengt: schmutzig-braunes Öl, das beim Stehenlassen bei RT auskristallisierte. Das Kristallisat wird getrocknet, mit H₂O ausgerührt, abfiltriert und getrocknet. Ausbeute: 240 mg (52%). ¹H-NMR (CDCl₃): δ (ppm) = 8,29 (d, 1H, J=2,2 Hz, C2-H); 8,02 (dd, 1H, J=8,5 Hz, J=2,2 Hz, C6-H); 7,05 (d, 1H, J=8,5 Hz, C5-H); 2,38 (s, 3H, Methyl); Carboxyl-OH und Phenol-OH nicht sichtbar
¹³C-NMR ([D₆]DMSO): δ (ppm) = 167,28; 158,10; 128,01; 127,03; 125,47; 122,37; 114,00

### e) 4-Hydroxymethyl-2-methylthio-phenol

LiAlH₄ 95% (0,55 g, 14 mmol) wird in einem ausgeheizten und mit Argon gespülten Dreihalskolben in absolutes THF (10 ml) eingetragen. Unter Eiskühlung wird zu dieser Vorlage eine Lösung von 4-Hydroxy-3-methylthio-benzoesäure (1,37 g; 7,4 mmol) in absolutem THF (15 ml) innerhalb von 5 min. so zugetropft, daß nur mäßige Gasentwicklung erfolgt. Nach beendeter Zugabe wird die Kühlung entfernt und das Reaktionsgemisch 30 min bei RT und weitere 21 h bei 55-65°C gerührt. Nachdem das Reaktionsgemisch auf RT abgekühlt ist wird unter Eiskühlung vorsichtig mit Eiswasser versetzt. Der basische Al(OH)₃ Niederschlag wird durch Zusatz von 10%iger Schwefelsäure aufgelöst und die wäßrige, saure Lösung (pH=1) mit Diethylether (150 ml) extrahiert. Das phenolische Produkt wird aus dem vereinigten etherischen Extrakt mit 2 Portionen Natronlauge (10%, 50 ml) extrahiert. Der natronalkalische Auszug wird mit Salzsäure (20%ig) auf pH=7 eingestellt und der Niederschlag mit Diethylether (50 ml) aufgenommen, die neutrale wässrige Lösung mit Diethylether (100 ml) extrahiert. Der vereinte, etherische Extrakt wird mit gesättigter NaCl-Lösung gewaschen, über Na₂SO₄ getrocknet und eingeengt: es verbleibt ein kristalliner, weißer Rückstand. Ausbeute: 670 mg (57%).
¹H-NMR (CDCl₃): δ (ppm) = 7,50 (d, 1H, J=2,0 Hz, C3-H); 7,24 (dd, 1H, J=8,4 Hz, J=2,0 Hz, C5-H); 6,97 (d, 1H, J=8,3 Hz, C6-H); 4,60 (s, 2H, Methylen); 2,34 (s, 3H, Methyl); Hydroxyl-OH und Phenol-OH nicht sichtbar

### f) 5-(4-Fluorphenyl)-2-[(4'-hydroxy-3'-methylthio-phenyl)-methylthio]-4-(4-pyridyl)-1H-imidazol

5-(4-Fluorphenyl)-4-(4-pyridyl)-1H-imidazol-2-thion (200 mg, 0,7 mmol) wird in Eisessig (5 ml) suspendiert und durch Zusatz von konz. Salzsäure (10 Trpf.) in Lösung gebracht. In die tieforangefarbene Lösung wird 4-Hydroxymethyl-2-methylthiophenol (140 mg, 0,8 mmol) eingetragen. Das Reaktionsgemisch wird 2 h bei RT gerührt. Das Reaktionsgemisch wird mit dest. H₂O (5 ml) verdünnt und die wäßrige Lösung mit konz. Ammoniakwasser tropfenweise auf pH=8 eingestellt. Der orangefarbene Niederschlag wird 45 min bei RT *in situ* ausgerührt, abfiltriert, gründlich mit H₂O gewaschen und getrocknet, anschließend mit wenig Methanol digeriert. Der methanolische Überstand wird abfiltriert, der Rückstand nochmals mit Methanol gewaschen und getrocknet: schwach gelbliches Pulver, Ausbeute 140 mg (47%)
IR (KBr) : 1/λ[cm⁻¹] = 3423, 3099, 3057, 1600, 1500, 1417, 1227, 1159, 1019, 837, 829, 817, 799, 694, 577;
¹H-NMR ([D₃]MeOD): δ (ppm) = 8,41 (2H, J=4,8 Hz, J=1,4 Hz, AA' 4-Pyr); 7,5-7,3 (m, 4H, BB' 4-Pyr + 4-F-Ph); 7,21-7,12 (m, 2H, 4-F-Ph); 7,0-6,9 (m, 2H, C2'-H + C6'-H in 4'-OH-Ph); 6,69 (d, 1H, J=8,0 Hz, C5'-H in 4'-OH-Ph); 4,17 (s, 2H, Methylen); 2,21 (s, 3H, Methyl)

### Beispiel 12

### 5-(4-Fluorphenyl)-2-[(4-hydroxy-3-methylsulfinyl-phenyl)-methylthio]-4-(4-pyridyl)-1H-imidazol

5-(4-Fluorphenyl)-4-(4-pyridyl)-1H-imidazol-2-thion (200 mg, 0,7 mmol) wird in 5 ml Eisessig suspendiert und durch Zusatz von 15 Tropfen konz. Salzsäure in Lösung gebracht. In die orangefarbene Lösung wird 4-Hydroxymethyl-2-methylthio-phenol (140 mg, 0,8 mmol) portionsweise eingetragen. Das Reaktionsgemisch wird 2,5 h bei RT gerührt. Dem Reaktionsgemisch wird bei RT 35%ige H₂O₂-Lösung (0,1 ml, 1 mmol) zugetropft. Das Reaktionsgemisch wird weitere 4 h bei RT gerührt. Das Reaktionsgemisch wird mit 5 ml dest. H₂O verdünnt und die wäßrige Lösung mit konz. Ammoniakwasser tropfenweise auf pH=8 eingestellt. Der Niederschlag wird 15 min bei RT *in situ* ausgerührt, abfiltriert, gründlich mit H₂O gewaschen und getrocknet. Das Rohprodukt wird mit Aceton ausgerührt, abfiltriert und getrocknet: hellorangenes Pulver, Ausbeute 170 mg (55%)
IR (KBr) : 1/λ[cm⁻¹] = 3435, 3117, 3063, 2360, 2325, 1604, 1503, 1425, 1296, 1280, 1230, 1160, 1062, 1013, 999, 833, 818
¹H-NMR ([D₃]MeOD): δ (ppm) = 8,40 (dd, 2H, J=4,8 Hz, J=1,5 Hz, AA' 4-Pyr); 7,46-7,39 (m, 5H, BB' 4-Pyr, 4-F-Ph, C2'-H); 7,28 (dd, 1H, J=8,3 Hz, J=2,2 Hz, C6'-H); 7,21-7,12 (m, 2H, 4-F-Ph); 6,78 (d, 1H, J=8,3 Hz, C5'-H); 4,28 (s, 2H, Methylen); 2,70 (s, 3H, Methyl)

### Beispiel 13

### 2-[(5-Chlor-2-hydroxy-3-methylthio-phenyl)-methylthio]-5-(4-fluorphenyl)-4-(4-pyridyl)-1H-imidazol

Analog zu Beispiel 11 aus 5-Chlor-2-hydroxy-3-methylthio-benzylalkohol und 5-(4-Fluorphenyl)-4-(4-pyridyl)-1H-imidazol-2-thion in Eisessig / konz. Salzsäure:
¹H-NMR ([D₆]DMSO): δ (ppm) = 12,74 (bs, 1H, NH); 8,49 (m, 2H, AA' 4-Pyr).; 7,51-7,23 (m, 6H, BB' 4-Pyr, 4-F-Ph); 7,17 (d, 1H, J=2,3 Hz, C4'-H); 6,97 (d, 1H, J=2,3 Hz, C2'-H); 4,38 (s, 2H, Methylen); 2,34 (s, 3H, Methyl); Phenol-OH nicht sichtbar;
IR (KBr): 1/λ[(cm⁻¹] = 3057, 2919, 2643, 2517, 1604, 1511, 1419, 1259, 1225, 1007, 988, 834, 589

### Beispiel 14

### 2-[(2-Hydroxy-5-methylthio-phenyl)-methylthio]-5-(4-fluorphenyl)-4-(4-pyridyl)-1H-imidazol

Analog zu Beispiel 11 aus 2-Hydroxy-5-methylthio-benzylalkohol und 5-(4-Fluorphenyl)-4-(4-pyridyl)-1H-imidazol-2-thion in Eisessig / konz. Salzsäure:
¹H-NMR ([D₇]DMF): δ (ppm) = 10,7-10,3 (bs, 1H, austauschbar, NH); 8,54 (m, 2H, AA' 4-Pyr); 7,65-7,58 (m, 2H, 4-F-Ph); 7,52 (m, 2H, BB' 4-Pyr); 7,35-7,27 (m, 3H, 4-F-Ph + C2'-H); 7,13 (dd, 1H, J=8,3 Hz, J=2,3 Hz, C4'-H); 6,90 (d, 1H, J=8,4 Hz, C5'-H); 4,46 (s, 2H, Methylen); 2,36 (s, 3H, Methyl); Phenol-OH nicht sichtbar;
¹³C-NMR ([D_{7]}DMF): δ (ppm) = 165,6; 162,9; 160,7; 155,0; 150,7; 143,5; 131,6; 131,4; 131,2; 129,8; 127,6; 126,5; 121,6; 120,0; 117,5; 116,6; 116,2; 32,5; 17,5;
IR (KBr) 1/λ[cm⁻¹]: 3057, 2913, 2661, 2607, 1602, 1511, 1486, 1418, 1382, 1275, 1255, 1230, 1158, 1005, 990, 838, 817, 590, 529

### Beispiel 15

### 2-[(5-Chlor-2-hydroxy-3-methylsulfinyl-phenyl)-methylthio]-5-(4-fluorphenyl)-4-(4-pyridyl)-1H-imidazol

Analog zu Beispiel 12 aus 5-Chlor-2-hydroxy-3-methylthio-benzylalkohol und 5-(4-Fluorphenyl)-4-(4-pyridyl)-1H-imidazol-2-thion in Eisessig / konz. Salzsäure/H₂O₂:
¹H-NMR ([D₃]MeOD): δ (ppm) = 8,45 (m, 2H, AA' 4-Pyr); 7,49-7,42 (m, 6H, BB' 4-Pyr, 4-F-Ph, C4'-H); 7,39 (d, 1H, J=2,6 Hz, C2'-H); 7,23-7,14 (m, 2H, 4-F-Ph); 4,39 (s, 2H, Methylen); 2,72 (s, 3H, Methyl)
IR (KBr) : 1/λ(cm⁻¹] = 3420, 3057, 2997, 2925, 2823, 2655, 2517, 2457, 2360, 1605, 1509, 1416, 1392, 1265, 1236, 1158, 1086, 1051, 1005, 832, 595, 534

### Beispiel 16

### 2-[(2-Hydroxy-5-methylsulfinyl-phenyl)-methylthio]-5-(4-fluorphenyl)-4-(4-pyridyl)-1H-imidazol

Analog zu Beispiel 12 aus 2-Hydroxy-5-methylthio-benzylalkohol und 5-(4-Fluorphenyl)-4-(4-pyridyl)-1H-imidazol-2-thion in Eisessig / konz. Salzsäure/H₂O₂:
¹H-NMR ([D₃]MeOD): δ (ppm) = 8,41 (m, 2H, AA' 4-Pyr); 7,47-7,41 (m, 6H, BB' 4-Pyr, 4-F-Ph, C2'-H, C4'-H); 7,21-7,11 (m, 2H, 4-F-Ph); 6,96 (d, 1H, J=8,2 Hz, C5'-H); 4,33 (s, 2H, Methylen); 2,60 (s, 3H, Methyl);
¹³C-NMR ([D₃]MeOD): δ (ppm) = 166,87; 161,95; 160,11; 150,28; 143,07; 134,91; 132,01; 131,85; 128,58; 127,73; 127,72; 126,37; 122,99; 117,58; 117,22; 116,78; 43,45; 34,57
IR (KBr): 1/λ[cm⁻¹] = 3410, 3129, 3069, 2991, 2913, 2360, 1603, 1571, 1500, 1422, 1280, 1232, 1157, 1078, 1031, 1003, 826, 697, 586

### Aktivitätstests

### Testsystem zur Bestimmung der Hemmung der 5-Lipoxygenase (5-LO)

Als Quelle für die 5-Lipoxygenase dienen menschliche Granulozyten. Durch Stimulation mit Calcium-Ionophor A 23187 wird LTB₄ (Leukotrien B₄) aus endogener Arachidonsäure gebildet. Die Isolierung der Granulozyten und die Durchführung der Enzymreaktion erfolgt nach bekannten Verfahren (siehe Arch. Pharm. Pharm. Med. Chem. 330, 307 - 312 (1997)).

Das mit Heparin vor Gerinnung geschützte Blut wird über einem diskontinuierlichen Percoll®-Gradienten zentrifugiert und die Granulozytenschicht abpipettiert. Nach Lyse der Erythrozyten werden die Granulozyten mehrmals gewaschen und anschließend auf eine bestimmte Zellzahl eingestellt. Die Enzymreaktion wird dann in An- bzw. Abwesenheit der Testsubstanz nach Zugabe von Ca²⁺ mit Calcium-Ionophor A 23187 gestartet. Die Synthese der Leukotriene wird nach 1,5 Minuten gestoppt. Die Proben werden abzentrifugiert, der Überstand verdünnt. Die quantitative Bestimmung von LTB₄ erfolgt mittels ELISA.

### Testsystem zur Bestimmung der Hemmung der Cyclooxygenase-1 (COX-1)

Bei diesem Testsystem wird die von menschlichen Thrombozyten nach Zusatz von Calcium-Ionophor gebildete Prostaglandin-E₂-Menge mittels ELISA bestimmt. Dabei werden die Thrombozyten nach Zentrifugation über einen diskontinuierlichen Percoll®-Gradienten gewonnen. Die Enzymreaktion und die Bestimmung der gebildeten Metaboliten erfolgt prinzipiell wie bei der Bestimmung der 5-Lipoxygenase-Hemmung. Unterschiede bestehen hinsichtlich der Inkubationszeit.. Weiterhin ist die Zugabe eines Thromboxansynthase-Hemmstoffes notwendig (siehe Arch. Pharm. Pharm. Med. Chem. 330, 307 - 312 (1997)).

### Testsystem zur Bestimmung der Hemmung der Cyclooxygenase-2 (COX-2)

COX-2 (aus Placenta des Schafs) wird mit Testsubstanz 10 min bei 4°C vorinkubiert, dann mit Arachidonsäure (5 µM) bei 25°C 10 min. stimuliert. Als Referenz dient Diclofenac (IC₅₀(COX-2) = 3,0 10⁻⁶ M). Die Bestimmung erfolgt in 3 Verdünnungen (10⁻⁷, 10⁻⁶, 10⁻⁵ molar), Die PGE₂-Konzentrationen werden mittels ELISA quantifiziert (siehe Mitchell J.A. et al. Proc. Nat. Acad. Sci 90: 11693-11697 (1993)).

### Testsystem zur Bestimmung der Hemmung der LPS-stimulierten Cytokinsekretion (TNF-α, IL-1β)

Humane PBMC (peripheral blood mononuclear cells) werden mit Testsubstanz 5 min bei 37°C vorinkubiert, anschließend mit LPS (1µg/ml) für 24 h bei 37°C stimuliert. Die Bestimmung der Zytokine TNF-α, IL-1β, IL-6 und IL-8 erfolgt mittels ELISA (siehe Blood 75, 40-47 (1990)).

### Testsystem zur Bestimmung der Hemmung der LPS-stimulierten Cytokinsekretion (TNF-α, IL-1β) im Vollblut

Frisches, humanes Vollblut von gesunden Spendern wird mit Testsubstanz 25 min. bei 37°C vorinkubiert. Die Stimulation der Zellen erfolgt mit LPS (1µg/ml) für 4h bei 37°C. Im Plasmaüberstand, der durch Zentrifugation isoliert wird, werden die Zytokine TNF-α, IL-1β, IL-6 und IL-8 mittels ELISA quantifiziert (siehe Inflamm. Res. 44, 269-274 (1995)).

Die Ergebnisse der mit den vorstehend beschriebenen Testsystemen durchgeführten Aktivitätstests sind in Tabellen 1 und 2 zusammengefaßt. Die Tabellen zeigen den Einfluß der erfindungsgemäßen Verbindungen der Beispiele 1-7, 9, 10, 12 und 16 auf die Freisetzung der Entzündungsmediatoren COX-1, COX-2, 5-LO, TNF-α und IL-1β (Tabelle 1) im Vergleich zu den Referenzsubstanzen A und B (Tabelle 2), deren Struktur ebenfalls in der Tabelle 2 angegeben ist.

Die Referenzsubstanzen A und B wurden nach dem in WO 93/14081 (dort S. 35. Beispiele 19 und 20) beschriebenen Vorgehen synthetisiert.

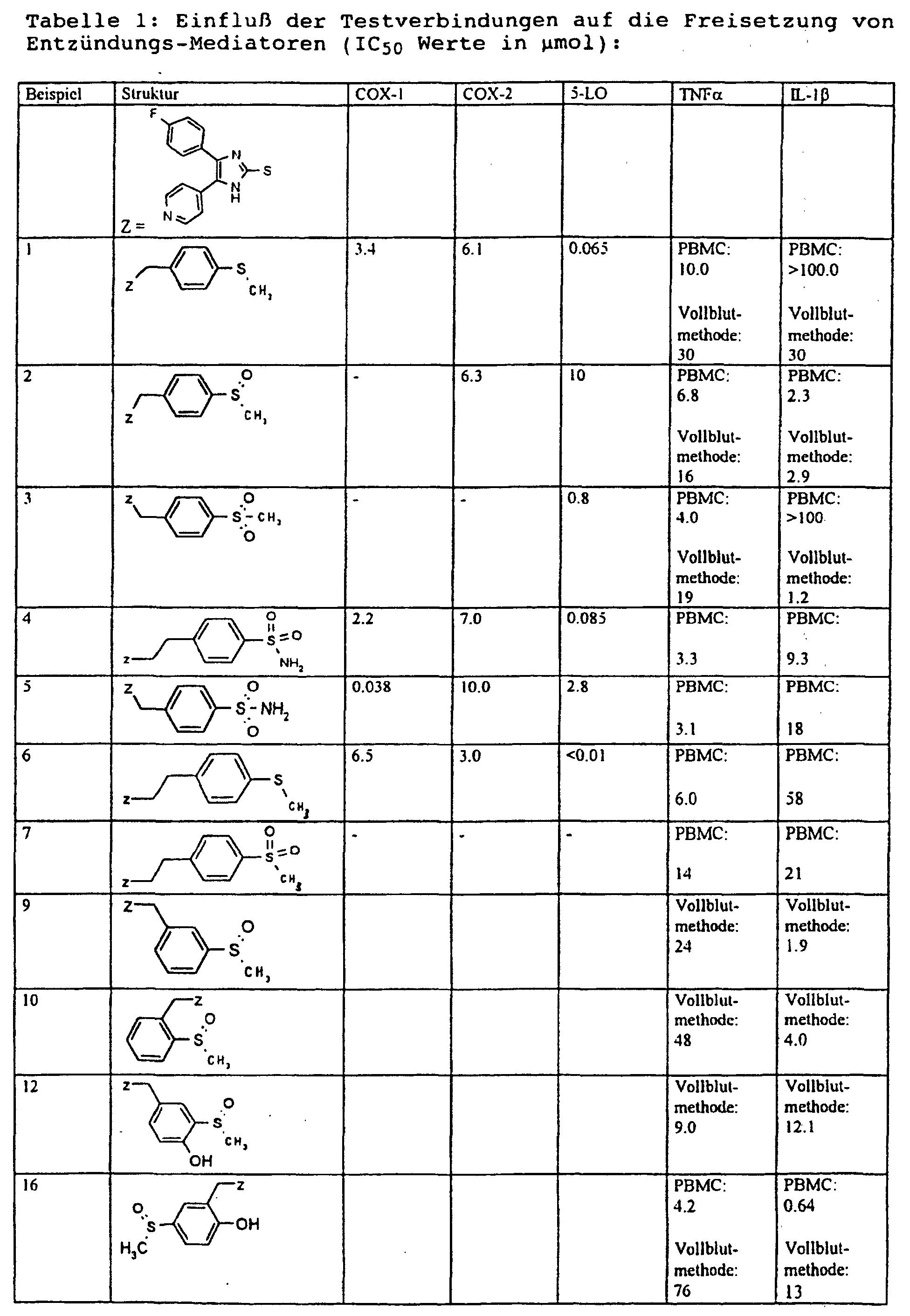

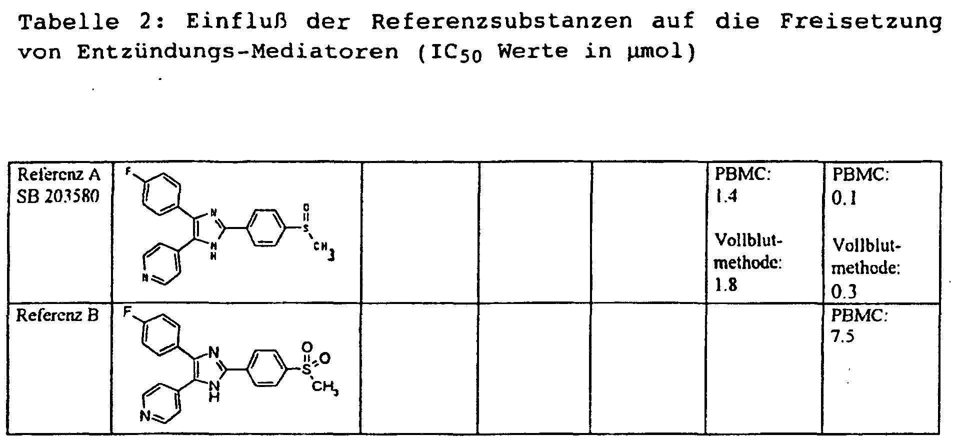

## Patentansprüche

1. Verbindung der allgemeinen Formel I 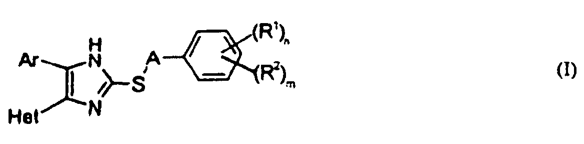 worin
Ar für einen Phenylrest steht, der gegebenenfalls mit einem oder mehreren Substituenten, ausgewählt aus Halogen, C₁₋₄-Alkyl, C₁₋₄-Alkoxy und C₁₋₄-Alkylthio, substituiert sein kann;
Het für einen Pyridyl-, Pyrimidinyl- oder Pyrazinylrest steht der gegebenenfalls mit einem oder mehreren Substituenten, ausgewählt aus Halogen, Amino, C₁₋₄-Alkylamino, C₁₋₄-Alkyl, Hydroxy, C₁₋₄-Alkoxy und C₁₋₄-Alkylthio, substituiert sein kann;
A für eine geradkettige oder verzweigte, gesättigte oder ungesättigte Alkylenkette mit bis zu 6-Kohlenstoffatomen steht;
R¹ für C₁₋₄-Alkylthio, C₁₋₄-Alkylsulfinyl, C₁₋₄-Alkylsulfonyl, Sulfonamido und C₁₋₄-Alkylcarbonyl steht;
R² für Halogen, C₁₋₄-Alkyl, Hydroxy, C₁₋₄-Alkoxy, C₁₋₄-Alkoxycarbonyl, Sulfonamido, Carboxyl, Nitro und Aminocarbonyl steht;
n 1 oder 2 ist und
m 0 bis 2 ist
oder ein pharmazeutisch verträgliches Salz davon.

2. Verbindung nach Anspruch 1, worin der heteroaromatische Rest Het eine 4-Pyridyl-, eine 3-Amino-4-pyridyl-, eine 2,4-Pyrimidinyl- oder eine 3-Amino-2,4-pyrimidinylgruppe ist.

3. Verbindung nach einem der vorhergehenden Ansprüche, worin der Phenylrest Ar mit einem oder mehreren Substituenten substituiert ist, ausgewählt aus Fluor, Chlor, Brom, C₁₋₄-Alkyl, C₁₋₄-Alkoxy, C₁₋₄-Alkylthio.

4. Verbindung nach Anspruch 3, worin der oder die Substituenten für Ar ausgewählt sind aus Fluor, Chlor, Methoxy und Methylthio.

5. Verbindung nach Anspruch 4 worin der Phenylrest Ar eine 4-Fluorphenylgruppe ist.

6. Verbindung nach einem der vorhergehenden Ansprüche, worin n 1 ist.

7. Verbindung nach einem der vorhergehenden Ansprüche, worin der Phenylrest Ar eine 4-Fluorphenylgruppe ist, der heteroaromatische Rest Het eine 4-Pyridylgruppe ist, A für Methylen oder Ethylen steht und n 1 ist.

8. Verbindung nach einem der vorhergehenden Ansprüche, ausgewählt aus
5-(4-Fluorphenyl)-2-[(4-methylsulfanyl-phenyl)-methylsulfanyl]-4-pyridyl-imidazol,
5-(4-Fluorphenyl)-2-[(4-methylsulfinyl-phenyl)-methylsulfanyl]-4-pyridyl-imidazol,
5-(4-Fluorphenyl)-2-[(4-methylsulfonyl-phenyl)-methylsulfanyl]-4-pyridyl-imidazol,
2-[(4-Aminosulfonyl-phenyl)-methylsulfanyl]-5-(4-fluorphenyl)-4-pyridyl-imidazol,
2-[2-(4-Aminosulfonyl-phenyl)-ethylsulfanyl)-5-(4-fluorphenyl)-4-pyridyl-imidazol,
5-(4-Fluorphenyl)-2-[2-(4-methylsulfanyl-phenyl)-ethylsulfanyl)-4-pyridyl-imidazol,
5-(4-Fluorphenyl)-2-(2-(4-methylsulfonyl-phenyl)-ethylsulfanyl)-4-pyridyl-imidazol,
5-(4-Fluorphenyl)-2-[(3-methylsulfanyl-phenyl)-methylsulfanyl]-4-pyridyl-imidazol,
5-(4-Fluorphenyl)-2-[(2-methylsulfanyl-phenyl)-methylsulfanyl]-4-pyridyl-imidazol,
5-(4-Fluorphenyl)-2-[(3-methylsulfinyl-phenyl)-methylsulfanyl]-4-pyridyl-imidazol,
5-(4-Fluorphenyl)-2-[(2-methylsulfinyl-phenyl)-methylsulfanyl]-4-pyridyl-imidazol,
5-(4-Fluorphenyl)-2-[(4-hydroxy-3-methylsulfanyl-phenyl)-methylsulfanyl]-4-pyridyl-imidazol,
5-(4-Fluorphenyl)-2-[(4-hydroxy-3-methylsulfanyl-phenyl)-methylsulfanyl)-4-pyridyl-imidazol,
2-[(5-Chlor-2-hydroxy-3-methylsulfanyl-phenyl)-methylsulfanyl]-5-(4-fluorphenyl)- 4-pyridyl-imidazol und
2-((5-Chlor-2-hydroxy-3-methylsulfinyl-phenyl)-methylsulfanyl]-5-(4-fluorphenyl)- 4-pyridyl-imidazol.

9. Verfahren zur Herstellung einer Verbindung der allgemeinen Formel I nach Anspruch 1, worin ein Imidazol-2-thion der allgemeinen Formel II 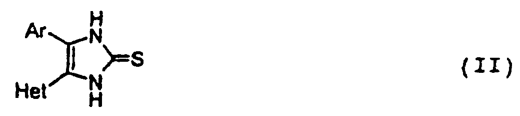 worin Ar und Het wie in Anspruch 1 definiert sind,
mit einer Verbindung der allgemeinen Formel III 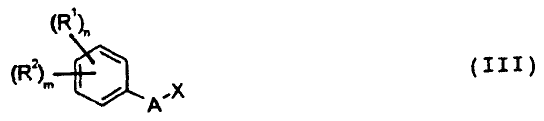 worin A, R¹, R², n und m wie in Anspruch 1 definiert sind und X eine Abgangsgruppe ist,
zu einer Verbindung der allgemeinen Formel I oder einem pharmazeutisch verträglichen Salz davon umgesetzt wird.

10. Arzneimittel, enthaltend eine verbindung der allgemeinen Formel I nach Anspruch 1 oder ein pharmazeutisch verträglichen Salz davon sowie gegebenenfalls übliche Trägerstoffe und Hilfsmittel.

## Claims

1. A compound of the general formula I 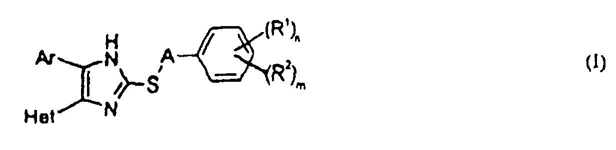 in which
Ar is a phenyl radical which can optionally be substituted by one or more substituents, selected from halogen, C₁₋₄-alkyl, C₁₋₄-alkoxy and C₁₋₄-alkylthio;
Het is a pyridyl, pyrimidinyl or pyrazinyl radical which can optionally be substituted by one or more substituents selected from halogen, amino, C₁₋₄-alkylamino, C₁₋₄-alkyl, hydroxyl, C₁₋₄-alkoxy and C₁₋₄-alkylthio;
A is a straight-chain or branched, saturated or unsaturated alkylene chain having up to 6 carbon atoms;
R¹ is C₁₋₄-alkylthio, C₁₋₄-alkylsulfinyl, C₁₋₄-alkylsulfonyl, sulfonamido or C₁₋₄-alkylcarbonyl;
R² is halogen , C₁₋₄-alkyl, hydroxyl, C₁₋₄-alkoxy, C₁₋₄-alkoxycarbonyl, sulfonamido, carboxyl, nitro or aminocarbonyl;
n is 1 or 2 and
m is 0 to 2
or a pharmaceutically tolerable salt thereof.

2. A compound as claimed in claim 1, in which the heteroaromatic radical Het is a 4-pyridyl, a 3-amino-4-pyridyl, a 2,4-pyrimidinyl or a 3-amino-2,4-pyrimidinyl group.

3. A compound as claimed in one of the preceding claims, in which the phenyl radical Ar is substituted by one or more substituents selected from fluorine, chlorine, bromine, C₁₋₄-alkyl, C₁₋₄-alkoxy, C₁₋₄-alkylthio.

4. A compound as claimed in claim 3, in which the substituent or the substituents for Ar are selected from fluorine, chlorine, methoxy and methylthio.

5. A compound as claimed in claim 4, in which the phenyl radical Ar is a 4-fluorophenyl group.

6. A compound as claimed in one of the preceding claims, in which n is 1.

7. A compound as claimed in one of the preceding claims, in which the phenyl radical Ar is a 4-fluorophenyl group, the heteroaromatic radical Het is a 4-pyridyl group, A is methylene or ethylene and n is 1.

8. A compound as claimed in one of the preceding claims, selected from
5-(4-fluorophenyl)-2-[(4-methylsulfanylphenyl)-methylsulfanyl]-4-pyridylimidazole,
5-(4-fluorophenyl)-2-[(4-methylsulfinylphenyl)-methylsulfanyl]-4-pyridylimidazole,
5-(4-fluorophenyl)-2-[(4-methylsulfonylphenyl)-methylsulfanyl]-4-pyridylimidazole,
2-[(4-aminosulfonylphenyl)methylsulfanyl]-5-(4-fluorophenyl)-4-pyridylimidazole,
2-[2-(4-aminosulfonylphenyl)ethylsulfanyl]-5-(4-fluorophenyl)-4-pyridylimidazole,
5-(4-fluorophenyl)-2-[2-(4-methylsulfanylphenyl)-ethylsulfanyl]-4-pyridylimidazole,
5-(4-fluorophenyl)-2-[2-(4-methylsulfonylphenyl)-ethylsulfanyl]-4-pyridylimidazole,
5-(4-fluorophenyl)-2-[(3-methylsulfanylphenyl)-methylsulfanyl]-4-pyridylimidazole,
5-(4-fluorophenyl)-2-[(2-methylsulfanylphenyl)-methylsulfanyl]-4-pyridylimidazole,
5-(4-fluorophenyl)-2-[(3-methylsulfinylphenyl)-methylsulfanyl]-4-pyridylimidazole,
5-(4-fluorophenyl)-2-[(2-methylsulfinylphenyl)-methylsulfanyl]-4-pyridylimidazole,
5-(4-fluorophenyl)-2-[(4-hydroxy-3-methylsulfanylphenyl)methylsulfanyl]-4-pyridylimidazole,
5-(4-fluorophenyl)-2-[(4-hydroxy-3-methylsulfanylphenyl)methylsulfanyl]-4-pyridylimidazole,
2-[(5-chloro-2-hydroxy-3-methylsulfanylphenyl)-methylsulfanyl]-5-(4-fluorophenyl)-4-pyridyl-imidazole and
2-[(5-chloro-2-hydroxy-3-methylsulfinylphenyl)-methylsulfanyl]-5-(4-fluorophenyl)-4-pyridyl-imidazole.

9. A process for the preparation of a compound of the general formula I as claimed in claim 1, in which an imidazole-2-thione of the general formula II 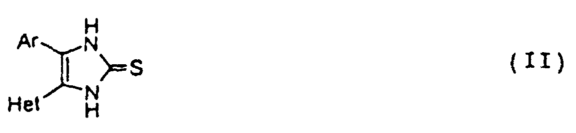 in which Ar and Het are as defined in claim 1,
is reacted with a compound of the general formula III 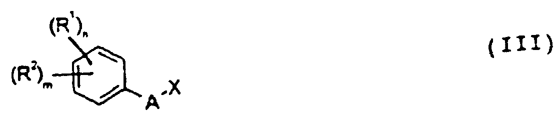 in which A, R¹, R², n and m are as defined in claim 1 and X is a leaving group,
to give a compound of the general formula I or a pharmaceutically tolerable salt thereof.

10. A medicament comprising a compound of the general formula I as claimed in claim 1 or a pharmaceutically tolerable salt thereof and, if appropriate, customary vehicles and excipients.

## Revendications

1. Composé de la formule générale I 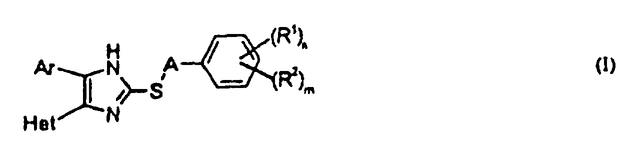 dans laquelle
Ar représente un reste phényle, qui peut éventuellement être substitué par un ou plusieurs substituants, choisis parmi un halogène, un alkyle en C₁ à C₄, un alcoxy en C₁ à C₄ et un alkylthio en C₁ à C₄,
Het représente un reste pyridyle, pyrimidinyle ou pyrazinyle, qui peut éventuellement être substitué par un ou plusieurs substituants, choisis parmi un halogène ou un groupe amino, (C₁-C₄)alkylamino, alkyle en C₁ à C₄, hydroxy, alcoxy en C₁ à C₄ et alkylthio en C₁ à C₄,
A représente une chaîne alkyle linéaire ou ramifiée, saturée ou non saturée, comportant jusqu'à 6 atomes de carbone,
R¹ représente un groupe (C₁-C₄)alkylthio, (C₁-C₄)alkylsulfinyle, (C₁-C₄)alkylsulfonyle, sulfonamido ou (C₁-C₄)alkylcarbonyle,
R² représente un halogène, ou un groupe alkyle en C₁à C₄, hydroxy, alcoxy en C₁ à C₄, (C₁-C₄)alcoxycarbonyle, sulfonamido, carboxyle, nitro ou aminocarbonyle,
n a une valeur de 1 ou 2 et
m a une valeur de 0 à 2,
ou un de leurs sels pharmaceutiquement compatibles.

2. Composé selon la revendication 1, dans lequel le reste hétéroaromatique Het est un groupe 4-pyridyle, 3-aminopyridyle, 2,4-pyrimidinyle ou 3-amino-2,4-pyrimidinyle.

3. Composé selon l'une quelconque des revendications qui précèdent, dans lequel le reste phényle Ar est substitué par un ou plusieurs substituants choisis parmi le fluor, le chlore, le brome, un alkyle en C₁ à C₄, un alcoxy en C₁ à C₄ et un alkylthio en C₁ à C₄.

4. Composé selon la revendication 3, dans lequel le ou les substituant(s) de Ar sont choisis parmi le fluor, le chlore, un méthoxy et un méthylthio.

5. Composé selon la revendication 4, dans lequel le reste phényle Ar est un groupe 4-fluorophényle.

6. Composé selon l'une quelconque des revendications qui précèdent, dans lequel n est égal à 1.

7. Composé selon l'une quelconque des revendications qui précèdent, dans lequel le reste phényle Ar est un groupe 4-fluorophényle, le reste hétéroaromatique Het est un groupe 4-pyridyle, A représente un méthylène ou un éthylène et n est égal à 1.

8. Composé selon l'une quelconque des revendications qui précèdent, choisi parmi:
le 5-(4-fluorophényl)-2-[(4-méthylthio-phényl)-méthylthio]-4-pyridyl-imidazole,
le 5-(4-fluorophényl)-2-((4-méthylsulfinyl-phényl)-méthylthio]-4-pyridyl-imidazole,
le 5-(4-fluorophényl)-2-[(4-méthylsulfonyl-phényl)-méthylthio]-4-pyridyl-imidazole,
le 2-[(4-aminosulfonyl-phényl)-méthylthio]-5-(4-fluorophényl)-4-pyridyl-imidazole,
le 2-[2-(4-aminosulfonyl-phényl)-éthylthio]-5-(4-fluorophényl)-4-pyridyl-imidazole,
le 5-(4-fluorophényl)-2-[2-(4-méthylthio-phényl)-éthylthio]-4-pyridyl-imidazole,
le 5-(4-fluorophényl)-2-[2-(4-méthylsulfonyl-phényl)-éthylthio)-4-pyridyl-imidazole,
le 5-(4-fluorophényl)-2-[(3-méthylthio-phényl)-méthylthio]-4-pyridyl-imidazole;
le 5-(4-fluorophényl)-2-[(2-méthylthio-phényl)-méthylthio]-4-pyridyl-imidazole,
le 5-(4-fluorophényl)-2-[(3-méthylsulfinyl-phényl)-méthylthio]-4-pyridyl-imidazole,
le 5-(4-fluorophényl)-2-[(2-méthylsulfinyl-phényl)-méthylthio]-4-pyridyl-imidazole,
le 5-(4-fluorophényl)-2-[(4-hydroxy-3-méthylthio-phényl)-méthylthio]-4-pyridyl-imidazole,
le 5-(4-fluorophényl)-2-[(4-hydroxy-3-méthylthio-phényl)-méthylthio]-4-pyridyl-irnidazole,
le 2-[(5-chloro-2-hydroxy-3-méthylthio-phényl)-méthylthio)-5-(4-fluorophényl)-4-pyridyl-imidazole,
le 2-[(5-chloro-2-hydroxy-3-méthylsulfinyl-phényl)-méthylthio]-5-(4-fluorophényl)-4-pyridyl-imidazole

9. Procédé de préparation d'un composé de la formule générale I selon la revendication 1, dans lequel on fait réagir une imidazole-2-thione de la formule générale II 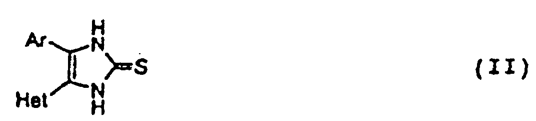 dans laquelle Ar et Het sont tels que définis dans la revendication 1, avec un composé de la formule générale III 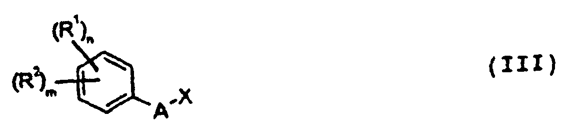 dans laquelle A, R¹, R², n et m sont tels que définis dans la revendication 1, et X est un groupe de départ, pour former un composé de la formule générale 1 ou l'un de sels pharmaceutiquement compatibles.

10. Médicaments contenant un composé de la formule générale I selon la revendication 1 ou l'un de sels pharmaceutiquement compatibles, ainsi qu'éventuellement des excipients et des adjuvants usuels.
